# EUROPÄISCHE PATENTSCHRIFT

(11) **EP 1 730 177 B1**
(45) Veröffentlichungstag und Bekanntmachung des Hinweises auf die Patenterteilung: **29.02.2012**
(21) Anmeldenummer: 05716033.5
(22) Anmeldetag: 14.03.2005
(51) Int. Cl.: C07K 14/21, C12P 13/02

(54) **CYANIDTOLERANTE NITRILHYDRATASEN**
CYANIDE TOLERANT NITRILHYDRATASES
NITRILHYDRATASES TOLERANTES AU CYANURE

(30) Priorität: 20.03.2004 DE 102004013847
(43) Veröffentlichungstag der Anmeldung: 13.12.2006
(73) Patentinhaber: Evonik Degussa GmbH, 45128 Essen (DE)
(72) Erfinder: OSSWALD, Steffen, 63517 Rodenbach (DE); WECKBECKER, Christoph, 63584 Gründau (DE); HUTHMACHER, Klaus, 63571 Gelnhausen (DE); GERASIMOVA, Tatijana, Moscow, 117545 (RU); NOVIKOV, Andrey, Moscow, 123610 (RU); RYABCHENKO, Ludmila, Odintcovo, 14311 (RU); YANENKO, Alexander, Moscow, 12193 (RU); EGOROVA, Ksenia, 21077 Hamburg (DE)
(86) Internationale Anmeldenummer: PCT/EP2005/002689
(87) Internationale Veröffentlichungsnummer: WO 2005/090394

(56) Entgegenhaltungen:
- EP-A- 1 266 962
- WO-A-98/37204
- WO-A-2004/067738
- WO-A-2004/111227
- US-A- 5 789 211
- BRADY D ET AL: "Characterisation of nitrilase and nitrile hydratase biocatalytic systems." APPLIED MICROBIOLOGY AND BIOTECHNOLOGY. MAR 2004, Bd. 64, Nr. 1, März 2004 (2004-03), Seiten 76-85, XP002333496 ISSN: 0175-7598
- NOJIRI M ET AL: "Functional expression of nitrile hydratase in Escherichia coli: requirement of a nitrile hydratase activator and post-translational modification of a ligand cysteine." JOURNAL OF BIOCHEMISTRY. APR 1999, Bd. 125, Nr. 4, April 1999 (1999-04), Seiten 696-704, XP002333497 ISSN: 0021-924X

### SEQUENCE LISTING

<110> Degussa AG
<120> Cyanidtolerante Nitrilhydratasen.
<130> 040061
<160> 14
<170> PatentIn version 3.3
<210> 1
   <211> 6828
   <212> DNA
   <213> Pseudomonas marginalis
<220>
   <221> CDS
   <222> (25)..(609)
   <223> Gen der Kodierregion der alpha-Untereinheit
<220>
   <221> CDS
   <222> (650)..(1312)
   <223> Gen der Kodierregion der beta-Unterinheit
<220>
   <221> gene
   <222> (1309)..(2577)
   <223> Gen des Aktivatorproteins
<400> 1
   aattcttaag aaggagatat acat atg agt aca gct act tca acg ccc ggc        51
                              Met Ser Thr Ala Thr Ser Thr Pro Gly
                              1               5
   gaa aga gcc tgg gca ttg ttt caa gtc ctc aag agc aag gaa ctc atc       99
   Glu Arg Ala Trp Ala Leu Phe Gln Val Leu Lys Ser Lys Glu Leu Ile
   10                  15                  20                  25
   ccg gag ggc tat gtc gag cag ctc acg caa ttg atg gag cac ggc tgg      147
   Pro Glu Gly Tyr Val Glu Gln Leu Thr Gln Leu Met Glu His Gly Trp
                   30                  35                  40
   agc ccc gag aac ggc gcc cgt gtg gtg gcc aag gcg tgg gtc gat ccg      195
   Ser Pro Glu Asn Gly Ala Arg Val Val Ala Lys Ala Trp Val Asp Pro
               45                  50                  55
   cag ttc cgg gca ctg ttg ctc aag gac ggc acc gcg gcc tgc gcc cag      243
   Gln Phe Arg Ala Leu Leu Leu Lys Asp Gly Thr Ala Ala Cys Ala Gln
           60                  65                  70
   ttc ggc tac acc ggc ccc cag ggc gaa tac atc gtt gcc ctg gag gat      291
   Phe Gly Tyr Thr Gly Pro Gln Gly Glu Tyr Ile Val Ala Leu Glu Asp
       75                  80                  85
   acg ccg acg ctg aag aac gtg att gtc tgc agc ctg tgc tcc tgc acc      339
   Thr Pro Thr Leu Lys Asn Val Ile Val Cys Ser Leu Cys Ser Cys Thr
   90                  95                  100                 105
   aac tgg ccg gtc ctc ggc ctg cca ccg gag tgg tac aag ggt ttc gag      387
   Asn Trp Pro Val Leu Gly Leu Pro Pro Glu Trp Tyr Lys Gly Phe Glu
                   110                 115                 120
   ttc cgc gca cgc ctg gtc cgg gag ggc cgc acg gta ctg cgc gag ctg      435
   Phe Arg Ala Arg Leu Val Arg Glu Gly Arg Thr Val Leu Arg Glu Leu
               125                 130                 135
   ggg acg gag ttg ccc cgg gac atg gtg gtc aag gtc tgg gac acc agc      483
   Gly Thr Glu Leu Pro Arg Asp Met Val Val Lys Val Trp Asp Thr Ser
           140                 145                 150
   gcc gaa agc cgc tac ctg gtg ctg ccg gtc agg ccg gaa ggc tca gaa      531
   Ala Glu Ser Arg Tyr Leu Val Leu Pro Val Arg Pro Glu Gly Ser Glu
       155                 160                 165
   cac atg agc gaa gag cag ctt caa gcg ctg gtg acc aaa gac gtg ctg      579
   His Met Ser Glu Glu Gln Leu Gln Ala Leu Val Thr Lys Asp Val Leu
   170                 175                 180                 185
   atc ggc gtc gcc ctg ccc cgc gtg ggc tga gaacaacacc tcatcatcgt        629
   Ile Gly Val Ala Leu Pro Arg Val Gly
                   190
   tcactcccgg agttttgatt atg gat ggc ttt cac gat ctc ggc ggt ttc caa    682
                         Met Asp Gly Phe His Asp Leu Gly Gly Phe Gln
                         195                 200                 205
   ggc ttt gga aaa gtc cct cac acc atc aac agc ctg agc tac aaa cag      730
   Gly Phe Gly Lys Val Pro His Thr Ile Asn Ser Leu Ser Tyr Lys Gln
                   210                 215                 220
   gtg ttc aag cag gac tgg gag cat ctg gcc tac agc ttg atg ttc atc      778
   Val Phe Lys Gln Asp Trp Glu His Leu Ala Tyr Ser Leu Met Phe Ile
               225                 230                 235
   ggt gcc gac cac ttg aaa aag ttc agc gtg gac gaa gtg cgt cac gcc      826
   Gly Ala Asp His Leu Lys Lys Phe Ser Val Asp Glu Val Arg His Ala
           240                 245                 250
   gtc gaa cgc ctg gat gtg cgc cag cat gtc ggc acc cag tac tac gaa      874
   Val Glu Arg Leu Asp Val Arg Gln His Val Gly Thr Gln Tyr Tyr Glu
       255                 260                 265
   cgc tac gtc atc gcg acc gcc acc ctg ctg gtc gaa acc ggc gtg atc      922
   Arg Tyr Val Ile Ala Thr Ala Thr Leu Leu Val Glu Thr Gly Val Ile
   270                 275                 280                 285
   acc cag gcg gag ctt gat cag gcc ttg ggc tcc cac ttc aag ctg gcg      970
   Thr Gln Ala Glu Leu Asp Gln Ala Leu Gly Ser His Phe Lys Leu Ala
                   290                 295                 300
   aat ccc gcc cat gcc gag ggc cgc ccg gcg att acg ggg cgg ccg ccc     1018
   Asn Pro Ala His Ala Glu Gly Arg Pro Ala Ile Thr Gly Arg Pro Pro
               305                 310                 315
   ttc gag gtg ggg gat cgg gtg gtg gtg cga gac gaa tat gtg gct gga     1066
   Phe Glu Val Gly Asp Arg Val Val Val Arg Asp Glu Tyr Val Ala Gly
           320                 325                 330
   cac atc cgc atg ccc gcc tac gtg cgc ggc aag gaa ggc gtg gtc ctg      1114
   His Ile Arg Met Pro Ala Tyr Val Arg Gly Lys Glu Gly Val Val Leu
       335                 340                 345
   cac cgc acg tca gag aaa tgg ccg ttc ccc gac gca atc ggg cat ggc      1162
   His Arg Thr Ser Glu Lys Trp Pro Phe Pro Asp Ala Ile Gly His Gly
   350                 355                 360                 365
   gat gta agc gca gcc cat caa ccc acc tac cac gtc gag ttc gcc gtg      1210
   Asp Val Ser Ala Ala His Gln Pro Thr Tyr His Val Glu Phe Ala Val
                   370                 375                 380
   aag gac ctg tgg gga gat gcc gcc gat gag ggt ttt gtg gtg gtc gac      1258
   Lys Asp Leu Trp Gly Asp Ala Ala Asp Glu Gly Phe Val Val Val Asp
               385                 390                 395
   ctg ttc gaa agc tac ctg gac aag gcc gcc ggc gcg cgc gcg gtg aac      1306
   Leu Phe Glu Ser Tyr Leu Asp Lys Ala Ala Gly Ala Arg Ala Val Asn
           400                 405                 410
   cca tga cagacggcgc ccaggcaagc cgactgccgg tgacggtcct ttcgggcttc      1362
   Pro
   ctcggcgccg gcaagaccac cctgctcaac cacatcctgc gcaatcgcga aggcctgcgc    1422
   gtggccgtca tcgtcaatga catgagcgaa gtcaatatcg atgccgaaga ggtgcagcgc    1482
   gatgtcgcgc tgcaccgtgg tcgcgatgag ctgatcgaga tgagcaacgg gtgcatctgc    1542
   tgcaccctgc gcgccgattt gctcgagcag atcagcatgc tcgcacgcca acagcgtttc    1602
   gattacctgc tgattgaatc cacggggatc tccgagccga tgccggtcgc ggagacgttc    1662
   gccttccttg acgctgatgg cttcagcctc agcgaactgg cgcgcctgga caccttggtg    1722
   acggtggtcg atggcagtcg tttccaggaa ctgctcgaat cgccgcacac cgttgaccag    1782
   gatgacgcca cgccagacgc acccaagcgc cacctggccg atctgctgat cgaacaggtg    1842
   gagtacgcca acgtcattct cgtcaataag ctggatctga tcgatgcagc gcagtatcag    1902
   gccgtgcagg cgatcctcac aggccttaac ccgacggcgc ggatcatgcc gatggcccac    1962
   ggtaacatcc catcagccag cctgctcggc acccatctgt ttgatttacc cagcctcgcg    2022
   gcgtcgccgg gctggatgcg gaaaatggag gcggcagacg cgccggcctc cgagtcggac    2082
   acctatggcg tgacgtcctg ggtgtaccgt gagcgcgcac ctttccaccc gcaacggttg    2142
   ctcgactttc tccagcagcc ctggtgcaac gggcggttgc tgcgcagcaa aggttacttc    2202
   tggcttgcca gccgccacct ggaaaccggc ctgctggtgc aaagcggcaa gcggttccag    2262
   tgggactatg tcgggcgctg gtggaacttc atcgagccgt cgcaatggcc ccgggacgaa    2322
   taccggctgc agggcatcag ggccaaatgg gacagcgtgg tcggcgactg ccggcaggag    2382
   ttggtgttta tcggccaggg cctcgacacc gacgcgttac agcgcgagct cgaccactgc    2442
   ctgctgagcg cccaggaaat cgccgccggc ccactggcct ggcaagcgct gccaggggcg    2502
   accgcctttg accgacagac ccttgcccgc cccccacaca gcccatggcg attgccccca 2562
   tttgatccga gatagaagct tctgttttgg cggatgagag aagattttca gcctgataca 2622
   gattaaatca gaacgcagaa gcggtctgat aaaacagaat ttgcctggcg gcagtagcgc 2682
   ggtggtccca cctgacccca tgccgaactc agaagtgaaa cgccgtagcg ccgatggtag 2742
   tgtggggtct ccccatgcga gagtagggaa ctgccaggca tcaaataaaa cgaaaggctc 2802
   agtcgaaaga ctgggccttt cgttttatct gttgtttgtc ggtgaacgct ctcctgagta 2862
   ggacaaatcc gccgggagcg gatttgaacg ttgcgaagca acggcccgga gggtggcggg 2922
   caggacgccc gccataaact gccaggcatc aaattaagca gaaggccatc ctgacggatg 2982
   gcctttttgc gtttctacaa actcttttgt ttatttttct aaatacattc aaatatgtat 3042
   ccgctcatga gacaataacc ctgataaatg cttcaataat attgaaaaag gaagagtatg 3102
   agtattcaac atttccgtgt cgcccttatt cccttttttg cggcattttg ccttcctgtt 3162
   tttgctcacc cagaaacgct ggtgaaagta aaagatgctg aagatcagtt gggtgcacga 3222
   gtgggttaca tcgaactgga tctcaacagc ggtaagatcc ttgagagttt tcgccccgaa 3282
   gaacgttttc caatgatgag cacttttaaa gttctgctat gtggcgcggt attatcccgt 3342
   gttgacgccg ggcaagagca actcggtcgc cgcatacact attctcagaa tgacttggtt 3402
   gagtactcac cagtcacaga aaagcatctt acggatggca tgacagtaag agaattatgc 3462
   agtgctgcca taaccatgag tgataacact gcggccaact tacttctgac aacgatcgga 3522
   ggaccgaagg agctaaccgc ttttttgcac aacatggggg atcatgtaac tcgccttgat 3582
   cgttgggaac cggagctgaa tgaagccata ccaaacgacg agcgtgacac cacgatgcct 3642
   gtagcaatgg caacaacgtt gcgcaaacta ttaactggcg aactacttac tctagcttcc 3702
   cggcaacaat taatagactg gatggaggcg gataaagttg caggaccact tctgcgctcg 3762
   gcccttccgg ctggctggtt tattgctgat aaatctggag ccggtgagcg tgggtctcgc 3822
   ggtatcattg cagcactggg gccagatggt aagccctccc gtatcgtagt tatctacacg 3882
   acggggagtc aggcaactat ggatgaacga aatagacaga tcgctgagat aggtgcctca 3942
   ctgattaagc attggtaact gtcagaccaa gtttactcat atatacttta gattgattta 4002
   aaacttcatt tttaatttaa aaggatctag gtgaagatcc tttttgataa tctcatgacc 4062
   aaaatccctt aacgtgagtt ttcgttccac tgagcgtcag accccgtaga aaagatcaaa 4122
   ggatcttctt gagatccttt ttttctgcgc gtaatctgct gcttgcaaac aaaaaaacca 4182
   ccgctaccag cggtggtttg tttgccggat caagagctac caactctttt tccgaaggta 4242
   actggcttca gcagagcgca gataccaaat actgtccttc tagtgtagcc gtagttaggc 4302
   caccacttca agaactctgt agcaccgcct acatacctcg ctctgctaat cctgttacca 4362
   gtggctgctg ccagtggcga taagtcgtgt cttaccgggt tggactcaag acgatagtta 4422
   ccggataagg cgcagcggtc gggctgaacg gggggttcgt gcacacagcc cagcttggag 4482
   cgaacgacct acaccgaact gagataccta cagcgtgagc tatgagaaag cgccacgctt 4542
   cccgaaggga gaaaggcgga caggtatccg gtaagcggca gggtcggaac aggagagcgc 4602
   acgagggagc ttccaggggg aaacgcctgg tatctttata gtcctgtcgg gtttcgccac 4662
   ctctgacttg agcgtcgatt tttgtgatgc tcgtcagggg ggcggagcct atggaaaaac 4722
   gccagcaacg cggccttttt acggttcctg gccttttgct ggccttttgc tcacatgttc 4782
   tttcctgcgt tatcccctga ttctgtggat aaccgtatta ccgcctttga gtgagctgat 4842
   accgctcgcc gcagccgaac gaccgagcgc agcgagtcag tgagcgagga agcggaagag 4902
   cgcctgatgc ggtattttct ccttacgcat ctgtgcggta tttcacaccg catatatggt 4962
   gcactctcag tacaatctgc tctgatgccg catagttaag ccagtataca ctccgctatc 5022
   gctacgtgac tgggtcatgg ctgcgccccg acacccgcca acacccgctg acgcgccctg 5082
   acgggcttgt ctgctcccgg catccgctta cagacaagct gtgaccgtct ccgggagctg 5142
   catgtgtcag aggttttcac cgtcatcacc gaaacgcgcg aggcagctgc ggtaaagctc 5202
   atcagcgtgg tcgtgaagcg attcacagat gtctgcctgt tcatccgcgt ccagctcgtt 5262
   gagtttctcc agaagcgtta atgtctggct tctgataaag cgggccatgt taagggcggt 5322
   tttttcctgt ttggtcactt gatgcctccg tgtaaggggg aatttctgtt catgggggta 5382
   atgataccga tgaaacgaga gaggatgctc acgatacggg ttactgatga tgaacatgcc 5442
   cggttactgg aacgttgtga gggtaaacaa ctggcggtat ggatgcggcg ggaccagaga 5502
   aaaatcactc agggtcaatg ccagcgcttc gttaatacag atgtaggtgt tccacagggt 5562
   agccagcagc atcctgcgat gcagatccgg aacataatgg tgcagggcgc tgacttccgc 5622
   gtttccagac tttacgaaac acggaaaccg aagaccattc atgttgttgc tcaggtcgca 5682
   gacgttttgc agcagcagtc gcttcacgtt cgctcgcgta tcggtgattc attctgctaa 5742
   ccagtaaggc aaccccgcca gcctagccgg gtcctcaacg acaggagcac gatcatgcgc 5802
   acccgtggcc aggacccaac gctgcccgag atgcgccgcg tgcggctgct ggagatggcg 5862
   gacgcgatgg atatgttctg ccaagggttg gtttgcgcat tcacagttct ccgcaagaat 5922
   tgattggctc caattcttgg agtggtgaat ccgttagcga ggtgccgccg gcttccattc 5982
   aggtcgaggt ggcccggctc catgcaccgc gacgcaacgc ggggaggcag acaaggtata 6042
   gggcggcgcg cctacaatcc atgccaaccc gttccatgtg ctcgccgagg cggcataaat 6102
   cgccgtgacg atcagcggtc cagtgatcga agttaggctg gtaagagccg cgagcgatcc 6162
   ttgaagctgt ccctgatggt cgtcatctac ctgcctggac agcatggcct gcaacgcggg 6222
   catcccgatg ccgccggaag cgagaagaat cataatgggg aaggccatcc agcctcgcgt 6282
   cgcgaacgcc agcaagacgt agcccagcgc gtcggccgcc atgccggcga taatggcctg 6342
   cttctcgccg aaacgtttgg tggcgggacc agtgacgaag gcttgagcga gggcgtgcaa 6402
   gattccgaat accgcaagcg acaggccgat catcgtcgcg ctccagcgaa agcggtcctc 6462
   gccgaaaatg acccagagcg ctgccggcac ctgtcctacg agttgcatga taaagaagac 6522
   agtcataagt gcggcgacga tagtcatgcc ccgcgcccac cggaaggagc tgactgggtt 6582
   gaaggctctc aagggcatcg gtcgacgctc tcccttatgc gactcctgca ttaggaagca 6642
   gcccagtagt aggttgaggc cgttgagcac cgccgccgca aggaatggtg catgcatcga 6702
   tcaccacaat tcagcaaatt gtgaacatca tcacgttcat ctttccctgg ttgccaatgg 6762
   cccattttcc tgtcagtaac gagaaggtcg cgaattcagg cgctttttag actggtcgta 6822
   atgaac 6828
<210> 2
   <211> 194
   <212> PRT
   <213> Pseudomonas marginalis
<400> 2
   Met Ser Thr Ala Thr Ser Thr Pro Gly Glu Arg Ala Trp Ala Leu Phe
   1 5 10 15
   Gln Val Leu Lys Ser Lys Glu Leu Ile Pro Glu Gly Tyr Val Glu Gln
   20 25 30
   Leu Thr Gln Leu Met Glu His Gly Trp Ser Pro Glu Asn Gly Ala Arg
   35 40 45
   Val Val Ala Lys Ala Trp Val Asp Pro Gln Phe Arg Ala Leu Leu Leu
   50 55 60
   Lys Asp Gly Thr Ala Ala Cys Ala Gln Phe Gly Tyr Thr Gly Pro Gln
   65 70 75 80
   Gly Glu Tyr Ile Val Ala Leu Glu Asp Thr Pro Thr Leu Lys Asn Val
   85 90 95
   Ile Val Cys Ser Leu Cys Ser Cys Thr Asn Trp Pro Val Leu Gly Leu
   100 105 110
   Pro Pro Glu Trp Tyr Lys Gly Phe Glu Phe Arg Ala Arg Leu Val Arg
   115 120 125
   Glu Gly Arg Thr Val Leu Arg Glu Leu Gly Thr Glu Leu Pro Arg Asp
   130 135 140
   Met Val Val Lys Val Trp Asp Thr Ser Ala Glu Ser Arg Tyr Leu Val
   145 150 155 160
   Leu Pro Val Arg Pro Glu Gly Ser Glu His Met Ser Glu Glu Gln Leu
   165 170 175
   Gln Ala Leu Val Thr Lys Asp Val Leu Ile Gly Val Ala Leu Pro Arg
   180 185 190
   Val Gly
<210> 3
   <211> 220
   <212> PRT
   <213> Pseudomonas marginalis
<400> 3
   Met Asp Gly Phe His Asp Leu Gly Gly Phe Gln Gly Phe Gly Lys Val
   1 5 10 15
   Pro His Thr Ile Asn Ser Leu Ser Tyr Lys Gln Val Phe Lys Gln Asp
   20 25 30
   Trp Glu His Leu Ala Tyr Ser Leu Met Phe Ile Gly Ala Asp His Leu
   35 40 45
   Lys Lys Phe Ser Val Asp Glu Val Arg His Ala Val Glu Arg Leu Asp
   50 55 60
   Val Arg Gln His Val Gly Thr Gln Tyr Tyr Glu Arg Tyr Val Ile Ala
   65 70 75 80
   Thr Ala Thr Leu Leu Val Glu Thr Gly Val Ile Thr Gln Ala Glu Leu
   85 90 95
   Asp Gln Ala Leu Gly Ser His Phe Lys Leu Ala Asn Pro Ala His Ala
   100 105 110
   Glu Gly Arg Pro Ala Ile Thr Gly Arg Pro Pro Phe Glu Val Gly Asp
   115 120 125
   Arg Val Val Val Arg Asp Glu Tyr Val Ala Gly His Ile Arg Met Pro
   130 135 140
   Ala Tyr Val Arg Gly Lys Glu Gly Val Val Leu His Arg Thr Ser Glu
   145                 150                 155                 160
   Lys Trp Pro Phe Pro Asp Ala Ile Gly His Gly Asp Val Ser Ala Ala
                   165                 170                 175
   His Gln Pro Thr Tyr His Val Glu Phe Ala Val Lys Asp Leu Trp Gly
               180                 185                 190
   Asp Ala Ala Asp Glu Gly Phe Val Val Val Asp Leu Phe Glu Ser Tyr
           195                 200                 205
   Leu Asp Lys Ala Ala Gly Ala Arg Ala Val Asn Pro
       210                 215                 220
<210> 4
   <211> 1269
   <212> DNA
   <213> Pseudomonas marginalis
<220>
   <221> CDS
   <222> (1)..(1269)
   <223> Gen der Kodierregion des Aktivatorproteins
<400> 4
   atg aca gac ggc gcc cag gca agc cga ctg ccg gtg acg gtc ctt tcg       48
   Met Thr Asp Gly Ala Gln Ala Ser Arg Leu Pro Val Thr Val Leu Ser
   1               5                   10                  15
   ggc ttc ctc ggc gcc ggc aag acc acc ctg ctc aac cac atc ctg cgc       96
   Gly Phe Leu Gly Ala Gly Lys Thr Thr Leu Leu Asn His Ile Leu Arg
               20                  25                  30
   aat cgc gaa ggc ctg cgc gtg gcc gtc atc gtc aat gac atg agc gaa      144
   Asn Arg Glu Gly Leu Arg Val Ala Val Ile Val Asn Asp Met Ser Glu
           35                  40                  45
   gtc aat atc gat gcc gaa gag gtg cag cgc gat gtc gcg ctg cac cgt      192
   Val Asn Ile Asp Ala Glu Glu Val Gln Arg Asp Val Ala Leu His Arg
       50                  55                  60
   ggt cgc gat gag ctg atc gag atg agc aac ggg tgc atc tgc tgc acc      240
   Gly Arg Asp Glu Leu Ile Glu Met Ser Asn Gly Cys Ile Cys Cys Thr
   65                  70                  75                  80
   ctg cgc gcc gat ttg ctc gag cag atc agc atg ctc gca cgc caa cag      288
   Leu Arg Ala Asp Leu Leu Glu Gln Ile Ser Met Leu Ala Arg Gln Gln
                   85                  90                  95
   cgt ttc gat tac ctg ctg att gaa tcc acg ggg atc tcc gag ccg atg      336
   Arg Phe Asp Tyr Leu Leu Ile Glu Ser Thr Gly Ile Ser Glu Pro Met
               100                 105                 110
   ccg gtc gcg gag acg ttc gcc ttc ctt gac gct gat ggc ttc agc ctc      384
   Pro Val Ala Glu Thr Phe Ala Phe Leu Asp Ala Asp Gly Phe Ser Leu
           115                 120                 125
   agc gaa ctg gcg cgc ctg gac acc ttg gtg acg gtg gtc gat ggc agt      432
   Ser Glu Leu Ala Arg Leu Asp Thr Leu Val Thr Val Val Asp Gly Ser
       130                 135                 140
   cgt ttc cag gaa ctg ctc gaa tcg ccg cac acc gtt gac cag gat gac      480
   Arg Phe Gln Glu Leu Leu Glu Ser Pro His Thr Val Asp Gln Asp Asp
   145                 150                 155                 160
   gcc acg cca gac gca ccc aag cgc cac ctg gcc gat ctg ctg atc gaa      528
   Ala Thr Pro Asp Ala Pro Lys Arg His Leu Ala Asp Leu Leu Ile Glu
                   165                 170                 175
   cag gtg gag tac gcc aac gtc att ctc gtc aat aag ctg gat ctg atc      576
   Gln Val Glu Tyr Ala Asn Val Ile Leu Val Asn Lys Leu Asp Leu Ile
               180                 185                 190
   gat gca gcg cag tat cag gcc gtg cag gcg atc ctc aca ggc ctt aac      624
   Asp Ala Ala Gln Tyr Gln Ala Val Gln Ala Ile Leu Thr Gly Leu Asn
           195                 200                 205
   ccg acg gcg cgg atc atg ccg atg gcc cac ggt aac atc cca tca gcc      672
   Pro Thr Ala Arg Ile Met Pro Met Ala His Gly Asn Ile Pro Ser Ala
       210                 215                 220
   agc ctg ctc ggc acc cat ctg ttt gat tta ccc agc ctc gcg gcg tcg      720
   Ser Leu Leu Gly Thr His Leu Phe Asp Leu Pro Ser Leu Ala Ala Ser
   225                 230                 235                 240
   ccg ggc tgg atg cgg aaa atg gag gcg gca gac gcg ccg gcc tcc gag      768
   Pro Gly Trp Met Arg Lys Met Glu Ala Ala Asp Ala Pro Ala Ser Glu
                   245                 250                 255
   tcg gac acc tat ggc gtg acg tcc tgg gtg tac cgt gag cgc gca cct      816
   Ser Asp Thr Tyr Gly Val Thr Ser Trp Val Tyr Arg Glu Arg Ala Pro
               260                 265                 270
   ttc cac ccg caa cgg ttg ctc gac ttt ctc cag cag ccc tgg tgc aac      864
   Phe His Pro Gln Arg Leu Leu Asp Phe Leu Gln Gln Pro Trp Cys Asn
           275                 280                 285
   ggg cgg ttg ctg cgc agc aaa ggt tac ttc tgg ctt gcc agc cgc cac      912
   Gly Arg Leu Leu Arg Ser Lys Gly Tyr Phe Trp Leu Ala Ser Arg His
       290                 295                 300
   ctg gaa acc ggc ctg ctg gtg caa agc ggc aag cgg ttc cag tgg gac      960
   Leu Glu Thr Gly Leu Leu Val Gln Ser Gly Lys Arg Phe Gln Trp Asp
   305                 310                 315                 320
   tat gtc ggg cgc tgg tgg aac ttc atc gag ccg tcg caa tgg ccc cgg     1008
   Tyr Val Gly Arg Trp Trp Asn Phe Ile Glu Pro Ser Gln Trp Pro Arg
                   325                 330                 335
   gac gaa tac cgg ctg cag ggc atc agg gcc aaa tgg gac agc gtg gtc     1056
   Asp Glu Tyr Arg Leu Gln Gly Ile Arg Ala Lys Trp Asp Ser Val Val
               340                 345                 350
   ggc gac tgc cgg cag gag ttg gtg ttt atc ggc cag ggc ctc gac acc      1104
   Gly Asp Cys Arg Gln Glu Leu Val Phe Ile Gly Gln Gly Leu Asp Thr
           355                 360                 365
   gac gcg tta cag cgc gag ctc gac cac tgc ctg ctg agc gcc cag gaa      1152
   Asp Ala Leu Gln Arg Glu Leu Asp His Cys Leu Leu Ser Ala Gln Glu
       370                 375                 380
   atc gcc gcc ggc cca ctg gcc tgg caa gcg ctg cca ggg gcg acc gcc      1200
   Ile Ala Ala Gly Pro Leu Ala Trp Gln Ala Leu Pro Gly Ala Thr Ala
   385                 390                 395                 400
   ttt gac cga cag acc ctt gcc cgc ccc cca cac agc cca tgg cga ttg      1248
   Phe Asp Arg Gln Thr Leu Ala Arg Pro Pro His Ser Pro Trp Arg Leu
                   405                 410                 415
   ccc cca ttt gat ccg aga tag                                          1269
   Pro Pro Phe Asp Pro Arg
               420
<210> 5
   <211> 422
   <212> PRT
   <213> Pseudomonas marginalis
<400> 5
   Met Thr Asp Gly Ala Gln Ala Ser Arg Leu Pro Val Thr Val Leu Ser
   1               5                   10                  15
   Gly Phe Leu Gly Ala Gly Lys Thr Thr Leu Leu Asn His Ile Leu Arg
               20                  25                  30
   Asn Arg Glu Gly Leu Arg Val Ala Val Ile Val Asn Asp Met Ser Glu
           35                  40                  45
   Val Asn Ile Asp Ala Glu Glu Val Gln Arg Asp Val Ala Leu His Arg
       50                  55                  60
   Gly Arg Asp Glu Leu Ile Glu Met Ser Asn Gly Cys Ile Cys Cys Thr
   65                  70                  75                  80
   Leu Arg Ala Asp Leu Leu Glu Gln Ile Ser Met Leu Ala Arg Gln Gln
                   85                  90                  95
   Arg Phe Asp Tyr Leu Leu Ile Glu Ser Thr Gly Ile Ser Glu Pro Met
               100                 105                 110
   Pro Val Ala Glu Thr Phe Ala Phe Leu Asp Ala Asp Gly Phe Ser Leu
           115                 120                 125
   Ser Glu Leu Ala Arg Leu Asp Thr Leu Val Thr Val Val Asp Gly Ser
   130 135 140
   Arg Phe Gln Glu Leu Leu Glu Ser Pro His Thr Val Asp Gln Asp Asp
   145 150 155 160
   Ala Thr Pro Asp Ala Pro Lys Arg His Leu Ala Asp Leu Leu Ile Glu
   165 170 175
   Gln Val Glu Tyr Ala Asn Val Ile Leu Val Asn Lys Leu Asp Leu Ile
   180 185 190
   Asp Ala Ala Gln Tyr Gln Ala Val Gln Ala Ile Leu Thr Gly Leu Asn
   195 200 205
   Pro Thr Ala Arg Ile Met Pro Met Ala His Gly Asn Ile Pro Ser Ala
   210 215 220
   Ser Leu Leu Gly Thr His Leu Phe Asp Leu Pro Ser Leu Ala Ala Ser
   225 230 235 240
   Pro Gly Trp Met Arg Lys Met Glu Ala Ala Asp Ala Pro Ala Ser Glu
   245 250 255
   Ser Asp Thr Tyr Gly Val Thr Ser Trp Val Tyr Arg Glu Arg Ala Pro
   260 265 270
   Phe His Pro Gln Arg Leu Leu Asp Phe Leu Gln Gln Pro Trp Cys Asn
   275 280 285
   Gly Arg Leu Leu Arg Ser Lys Gly Tyr Phe Trp Leu Ala Ser Arg His
   290 295 300
   Leu Glu Thr Gly Leu Leu Val Gln Ser Gly Lys Arg Phe Gln Trp Asp
   305 310 315 320
   Tyr Val Gly Arg Trp Trp Asn Phe Ile Glu Pro Ser Gln Trp Pro Arg
   325 330 335
   Asp Glu Tyr Arg Leu Gln Gly Ile Arg Ala Lys Trp Asp Ser Val Val
   340 345 350
   Gly Asp Cys Arg Gln Glu Leu Val Phe Ile Gly Gln Gly Leu Asp Thr
   355 360 365
   Asp Ala Leu Gln Arg Glu Leu Asp His Cys Leu Leu Ser Ala Gln Glu
   370 375 380
   Ile Ala Ala Gly Pro Leu Ala Trp Gln Ala Leu Pro Gly Ala Thr Ala
   385                 390                 395                 400
   Phe Asp Arg Gln Thr Leu Ala Arg Pro Pro His Ser Pro Trp Arg Leu
                   405                 410                 415
   Pro Pro Phe Asp Pro Arg
               420
<210> 6
   <211> 2371
   <212> DNA
   <213> Pseudomonas putida
<220>
   <221> CDS
   <222> (1)..(582)
   <223> Gen der Kodierregion der alpha-Untereinheit
<220>
   <221> CDS
   <222> (624)..(1286)
   <223> Gen der Kodierregion der beta-Untereinheit
<220>
   <221> gene
   <222> (1283)..(2371)
   <223> Gen des Aktivatorproteins
<400> 6
   atg acg gca act tca acc cct ggt gag cgg gca cgc gca ttg ttt gca      48
   Met Thr Ala Thr Ser Thr Pro Gly Glu Arg Ala Arg Ala Leu Phe Ala
   1               5                   10                  15
   gtg ctc aag cgc aaa gac ctc atc ccc gag ggc tac atc gaa cag ctc      96
   Val Leu Lys Arg Lys Asp Leu Ile Pro Glu Gly Tyr Ile Glu Gln Leu
               20                  25                  30
   acc cag ctg atg gaa cac ggc tgg agc ccg gaa aac ggc gcg cgc atc     144
   Thr Gln Leu Met Glu His Gly Trp Ser Pro Glu Asn Gly Ala Arg Ile
           35                  40                  45
   gtc gcc aag gcc tgg gtc gat ccg cag ttt cgc gag ctg ctg ctc aag     192
   Val Ala Lys Ala Trp Val Asp Pro Gln Phe Arg Glu Leu Leu Leu Lys
       50                  55                  60
   gac ggt acg gcc gcc tgc gcc cag ttc ggc ttc acc ggc cca caa ggc     240
   Asp Gly Thr Ala Ala Cys Ala Gln Phe Gly Phe Thr Gly Pro Gln Gly
   65                  70                  75                  80
   gaa tac atc gtc gcc ctg gaa gac acc ccg cag ttg aaa aac gtg atc     288
   Glu Tyr Ile Val Ala Leu Glu Asp Thr Pro Gln Leu Lys Asn Val Ile
                   85                  90                  95
   gtc tgt agc ctg tgc tcc tgc acg aac tgg ccg gtg ctg ggc ctg cca     336
   Val Cys Ser Leu Cys Ser Cys Thr Asn Trp Pro Val Leu Gly Leu Pro
               100                 105                 110
   cct gag tgg tac aag ggc ttc gag ttc cgt gcg cgg ttg gtc cgg gag      384
   Pro Glu Trp Tyr Lys Gly Phe Glu Phe Arg Ala Arg Leu Val Arg Glu
           115                 120                 125
   ggg cgc acg gta ttg cgc gag ctg ggc acc gag ttg ccc ggc gac atg      432
   Gly Arg Thr Val Leu Arg Glu Leu Gly Thr Glu Leu Pro Gly Asp Met
       130                 135                 140
   gtg gtc aag gtc tgg gac acc agc gct gaa agc cgc tac ctg gtg ctg      480
   Val Val Lys Val Trp Asp Thr Ser Ala Glu Ser Arg Tyr Leu Val Leu
   145                 150                 155                 160
   ccg caa cga cca gcg ggc tca gag cat atg agc gaa gag cag ttg cgg      528
   Pro Gln Arg Pro Ala Gly Ser Glu His Met Ser Glu Glu Gln Leu Arg
                   165                 170                 175
   caa ctg gtc acc aag gac gtg ctg atc ggc gtc gcc ctg ccc cgc gtt      576
   Gln Leu Val Thr Lys Asp Val Leu Ile Gly Val Ala Leu Pro Arg Val
               180                 185                 190
   ggc tga gcaaggccgc ccaaccccat tcaacttccg gagtgttcaa t atg gat ggc    632
   Gly                                                   Met Asp Gly
                                                             195
   ttt cac gat ctc ggc ggt ttc cag ggc ttt ggc aaa gtg ccc cac cgc      680
   Phe His Asp Leu Gly Gly Phe Gln Gly Phe Gly Lys Val Pro His Arg
               200                 205                 210
   atc aac agc ctg agc tac aag cag gtg ttc aag cag gac tgg gaa cac      728
   Ile Asn Ser Leu Ser Tyr Lys Gln Val Phe Lys Gln Asp Trp Glu His
           215                 220                 225
   ctg gcc tac agc ctg atg ttc atc ggc gtc gac cac ctg aac aag ttc      776
   Leu Ala Tyr Ser Leu Met Phe Ile Gly Val Asp His Leu Asn Lys Phe
       230                 235                 240
   agc gtc gac gaa ata cgt cat gcc gtc gaa cgc att gac gtg cgc cag      824
   Ser Val Asp Glu Ile Arg His Ala Val Glu Arg Ile Asp Val Arg Gln
   245                 250                 255                 260
   cac gtc ggc acc gaa tac tac gaa cgt tat gtg atc gcc act gcc acg      872
   His Val Gly Thr Glu Tyr Tyr Glu Arg Tyr Val Ile Ala Thr Ala Thr
                   265                 270                 275
   ctg ctg gtc gaa aca ggc gtc atc acc cag gcc gaa ctg gat gaa gca      920
   Leu Leu Val Glu Thr Gly Val Ile Thr Gln Ala Glu Leu Asp Glu Ala
               280                 285                 290
   ctc ggc tcg cac ttc aag ctg gcc aac ccc gcc cat gcg caa ggg cgt      968
   Leu Gly Ser His Phe Lys Leu Ala Asn Pro Ala His Ala Gln Gly Arg
           295                 300                 305
   gct gca att atc ggg cga gcg cct ttt gaa gtg ggc gat cgg gtc atc     1016
   Ala Ala Ile Ile Gly Arg Ala Pro Phe Glu Val Gly Asp Arg Val Ile
       310                 315                 320
   gta cgc gat gaa tac gtg gcc ggg cat gtg cgc atg cct gca tac gtg     1064
   Val Arg Asp Glu Tyr Val Ala Gly His Val Arg Met Pro Ala Tyr Val
   325                 330                 335                 340
   cgc ggc aag caa ggc gta gtg ctg cac cgg acc act gaa cag tgg ccg      1112
   Arg Gly Lys Gln Gly Val Val Leu His Arg Thr Thr Glu Gln Trp Pro
                   345                 350                 355
   ttt ccg gac gcg att ggc cat ggc gac cag agc gct gcg cat caa ccg      1160
   Phe Pro Asp Ala Ile Gly His Gly Asp Gln Ser Ala Ala His Gln Pro
               360                 365                 370
   acc tac cat gtc gag ttc cgc gtg cgg gac ctg tgg ggc gat gcc gca      1208
   Thr Tyr His Val Glu Phe Arg Val Arg Asp Leu Trp Gly Asp Ala Ala
           375                 380                 385
   gac gac ggc ctg gtg gtg gta gac ctg ttc gaa agc tat ctg gac agg      1256
   Asp Asp Gly Leu Val Val Val Asp Leu Phe Glu Ser Tyr Leu Asp Arg
       390                 395                 400
   gtc gaa agc ccg cga gtg gtg cgc gca tga gtgccggcgc ccaggcaggc        1306
   Val Glu Ser Pro Arg Val Val Arg Ala
   405                 410
   cggctgccgg tgacggtcct ttcaggcttc ctcggcgcag gcaagaccac cctgctcaac    1366
   cacatcctgc gcaaccgcca gggcctgaag gtggcggtta tcgtcaatga catgagcgag    1426
   gtcaacatcg atgccgccca ggtccagcgc gacgttgcgc tgtatcgtgg ccaggatgaa    1486
   ttgatagaga tgagcaacgg ctgtatctgc tgcaccctgc gcgccgacct gcttgagcag    1546
   atcagcgcgc tggcgcgcca gcagcgtttc gattacctgt tgatcgagtc caccgggatt    1606
   tccgagccga tgccagtcgc cgagaccttt gcctttctcg acgccaacgg tttcagcctc    1666
   agcgaactgg cgcggctgga tacgctggtg acggtggtcg atgccagcca gttcatggcc    1726
   atgctcgact ctcccgaaac cgtcgcgcgg gccgacgtca ccacggatga cagcaggcgc    1786
   ccgctggccg atctgctgat cgagcaggtc gagtatgcca atgtgattct ggtcaacaaa    1846
   cgcgacctgg tcgacgaggc gcagtaccag gccctgcagg cagttctcgc cgggctcaat    1906
   ccaggcgcac agatcctgcc gatggtggcc ggcaacgtcg ccctgtcgag cgtccttggt    1966
   acccagctgt tcgatttgcc cagccttgcc gcagcgcccg gctggatgaa acagatggac    2026
   gcgcacgaca ccccggccgg cgagtcgcag acctatggcg tgacgtcatg ggtgtaccga    2086
   gcgcgcgccc cgttccatcc gcaacgcttg cttgattttc tcgcccggcc ctggcgcgac    2146
   ggccgtcttc tgcgcagcaa aggttatttc tggcttgcca gccgccaccg cgaaatcggc    2206
   ttgctggtac acagcggcca gcagtttcaa tgggactatg ttggccattg gtggaacttc    2266
   atcgacacgt cacagtggcc acaggacaag tatcgcttgc agggcatcat ggccaagtgg    2326
   gacagcatcg tcggcgactg ccgacaggag ctgaaaagct tatga                    2371
<210> 7
   <211> 193
   <212> PRT
   <213> Pseudomonas putida
<400> 7
   Met Thr Ala Thr Ser Thr Pro Gly Glu Arg Ala Arg Ala Leu Phe Ala
   1               5                   10                  15
   Val Leu Lys Arg Lys Asp Leu Ile Pro Glu Gly Tyr Ile Glu Gln Leu
               20                  25                  30
   Thr Gln Leu Met Glu His Gly Trp Ser Pro Glu Asn Gly Ala Arg Ile
           35                  40                  45
   Val Ala Lys Ala Trp Val Asp Pro Gln Phe Arg Glu Leu Leu Leu Lys
       50                  55                  60
   Asp Gly Thr Ala Ala Cys Ala Gln Phe Gly Phe Thr Gly Pro Gln Gly
   65                  70                  75                  80
   Glu Tyr Ile Val Ala Leu Glu Asp Thr Pro Gln Leu Lys Asn Val Ile
                   85                  90                  95
   Val Cys Ser Leu Cys Ser Cys Thr Asn Trp Pro Val Leu Gly Leu Pro
               100                 105                 110
   Pro Glu Trp Tyr Lys Gly Phe Glu Phe Arg Ala Arg Leu Val Arg Glu
           115                 120                 125
   Gly Arg Thr Val Leu Arg Glu Leu Gly Thr Glu Leu Pro Gly Asp Met
       130                 135                 140
   Val Val Lys Val Trp Asp Thr Ser Ala Glu Ser Arg Tyr Leu Val Leu
   145                 150                 155                 160
   Pro Gln Arg Pro Ala Gly Ser Glu His Met Ser Glu Glu Gln Leu Arg
                   165                 170                 175
   Gln Leu Val Thr Lys Asp Val Leu Ile Gly Val Ala Leu Pro Arg Val
               180                 185                 190
   Gly
<210> 8
   <211> 220
   <212> PRT
   <213> Pseudomonas putida
<400> 8
   Met Asp Gly Phe His Asp Leu Gly Gly Phe Gln Gly Phe Gly Lys Val
   1               5                   10                  15
   Pro His Arg Ile Asn Ser Leu Ser Tyr Lys Gln Val Phe Lys Gln Asp
               20                  25                  30
   Trp Glu His Leu Ala Tyr Ser Leu Met Phe Ile Gly Val Asp His Leu
           35                  40                  45
   Asn Lys Phe Ser Val Asp Glu Ile Arg His Ala Val Glu Arg Ile Asp
       50                  55                  60
   Val Arg Gln His Val Gly Thr Glu Tyr Tyr Glu Arg Tyr Val Ile Ala
   65                  70                  75                  80
   Thr Ala Thr Leu Leu Val Glu Thr Gly Val Ile Thr Gln Ala Glu Leu
                   85                  90                  95
   Asp Glu Ala Leu Gly Ser His Phe Lys Leu Ala Asn Pro Ala His Ala
               100                 105                 110
   Gln Gly Arg Ala Ala Ile Ile Gly Arg Ala Pro Phe Glu Val Gly Asp
           115                 120                 125
   Arg Val Ile Val Arg Asp Glu Tyr Val Ala Gly His Val Arg Met Pro
       130                 135                 140
   Ala Tyr Val Arg Gly Lys Gln Gly Val Val Leu His Arg Thr Thr Glu
   145                 150                 155                 160
   Gln Trp Pro Phe Pro Asp Ala Ile Gly His Gly Asp Gln Ser Ala Ala
                   165                 170                 175
   His Gln Pro Thr Tyr His Val Glu Phe Arg Val Arg Asp Leu Trp Gly
               180                 185                 190
   Asp Ala Ala Asp Asp Gly Leu Val Val Val Asp Leu Phe Glu Ser Tyr
           195                 200                 205
   Leu Asp Arg Val Glu Ser Pro Arg Val Val Arg Ala
       210                 215                 220
<210> 9
   <211> 1089
   <212> DNA
   <213> Pseudomonas putida
<220>
   <221> CDS
   <222> (1)..(1089)
   <223> Gen der Kodierregion des Aktivatorproteins
<400> 9
   atg agt gcc ggc gcc cag gca ggc cgg ctg ccg gtg acg gtc ctt tca      48
   Met Ser Ala Gly Ala Gln Ala Gly Arg Leu Pro Val Thr Val Leu Ser
   1               5                   10                  15
   ggc ttc ctc ggc gca ggc aag acc acc ctg ctc aac cac atc ctg cgc      96
   Gly Phe Leu Gly Ala Gly Lys Thr Thr Leu Leu Asn His Ile Leu Arg
               20                  25                  30
   aac cgc cag ggc ctg aag gtg gcg gtt atc gtc aat gac atg agc gag      144
   Asn Arg Gln Gly Leu Lys Val Ala Val Ile Val Asn Asp Met Ser Glu
           35                  40                  45
   gtc aac atc gat gcc gcc cag gtc cag cgc gac gtt gcg ctg tat cgt      192
   Val Asn Ile Asp Ala Ala Gln Val Gln Arg Asp Val Ala Leu Tyr Arg
       50                  55                  60
   ggc cag gat gaa ttg ata gag atg agc aac ggc tgt atc tgc tgc acc      240
   Gly Gln Asp Glu Leu Ile Glu Met Ser Asn Gly Cys Ile Cys Cys Thr
   65                  70                  75                  80
   ctg cgc gcc gac ctg ctt gag cag atc agc gcg ctg gcg cgc cag cag      288
   Leu Arg Ala Asp Leu Leu Glu Gln Ile Ser Ala Leu Ala Arg Gln Gln
                   85                  90                  95
   cgt ttc gat tac ctg ttg atc gag tcc acc ggg att tcc gag ccg atg      336
   Arg Phe Asp Tyr Leu Leu Ile Glu Ser Thr Gly Ile Ser Glu Pro Met
               100                 105                 110
   cca gtc gcc gag acc ttt gcc ttt ctc gac gcc aac ggt ttc agc ctc      384
   Pro Val Ala Glu Thr Phe Ala Phe Leu Asp Ala Asn Gly Phe Ser Leu
           115                 120                 125
   agc gaa ctg gcg cgg ctg gat acg ctg gtg acg gtg gtc gat gcc agc      432
   Ser Glu Leu Ala Arg Leu Asp Thr Leu Val Thr Val Val Asp Ala Ser
       130                 135                 140
   cag ttc atg gcc atg ctc gac tct ccc gaa acc gtc gcg cgg gcc gac      480
   Gln Phe Met Ala Met Leu Asp Ser Pro Glu Thr Val Ala Arg Ala Asp
   145                 150                 155                 160
   gtc acc acg gat gac agc agg cgc ccg ctg gcc gat ctg ctg atc gag      528
   Val Thr Thr Asp Asp Ser Arg Arg Pro Leu Ala Asp Leu Leu Ile Glu
                   165                 170                 175
   cag gtc gag tat gcc aat gtg att ctg gtc aac aaa cgc gac ctg gtc      576
   Gln Val Glu Tyr Ala Asn Val Ile Leu Val Asn Lys Arg Asp Leu Val
               180                 185                 190
   gac gag gcg cag tac cag gcc ctg cag gca gtt ctc gcc ggg ctc aat      624
   Asp Glu Ala Gln Tyr Gln Ala Leu Gln Ala Val Leu Ala Gly Leu Asn
           195                 200                 205
   cca ggc gca cag atc ctg ccg atg gtg gcc ggc aac gtc gcc ctg tcg      672
   Pro Gly Ala Gln Ile Leu Pro Met Val Ala Gly Asn Val Ala Leu Ser
       210                 215                 220
   agc gtc ctt ggt acc cag ctg ttc gat ttg ccc agc ctt gcc gca gcg      720
   Ser Val Leu Gly Thr Gln Leu Phe Asp Leu Pro Ser Leu Ala Ala Ala
   225                 230                 235                 240
   ccc ggc tgg atg aaa cag atg gac gcg cac gac acc ccg gcc ggc gag      768
   Pro Gly Trp Met Lys Gln Met Asp Ala His Asp Thr Pro Ala Gly Glu
                   245                 250                 255
   tcg cag acc tat ggc gtg acg tca tgg gtg tac cga gcg cgc gcc ccg      816
   Ser Gln Thr Tyr Gly Val Thr Ser Trp Val Tyr Arg Ala Arg Ala Pro
               260                 265                 270
   ttc cat ccg caa cgc ttg ctt gat ttt ctc gcc cgg ccc tgg cgc gac      864
   Phe His Pro Gln Arg Leu Leu Asp Phe Leu Ala Arg Pro Trp Arg Asp
           275                 280                 285
   ggc cgt ctt ctg cgc agc aaa ggt tat ttc tgg ctt gcc agc cgc cac      912
   Gly Arg Leu Leu Arg Ser Lys Gly Tyr Phe Trp Leu Ala Ser Arg His
       290                 295                 300
   cgc gaa atc ggc ttg ctg gta cac agc ggc cag cag ttt caa tgg gac      960
   Arg Glu Ile Gly Leu Leu Val His Ser Gly Gln Gln Phe Gln Trp Asp
   305                 310                 315                 320
   tat gtt ggc cat tgg tgg aac ttc atc gac acg tca cag tgg cca cag     1008
   Tyr Val Gly His Trp Trp Asn Phe Ile Asp Thr Ser Gln Trp Pro Gln
                   325                 330                 335
   gac aag tat cgc ttg cag ggc atc atg gcc aag tgg gac agc atc gtc     1056
   Asp Lys Tyr Arg Leu Gln Gly Ile Met Ala Lys Trp Asp Ser Ile Val
               340                 345                 350
   ggc gac tgc cga cag gag ctg aaa agc tta tga                         1089
   Gly Asp Cys Arg Gln Glu Leu Lys Ser Leu
           355                 360
<210> 10
   <211> 362
   <212> PRT
   <213> Pseudomonas putida
<400> 10
   Met Ser Ala Gly Ala Gln Ala Gly Arg Leu Pro Val Thr Val Leu Ser
   1               5                   10                  15
   Gly Phe Leu Gly Ala Gly Lys Thr Thr Leu Leu Asn His Ile Leu Arg
               20                  25                  30
   Asn Arg Gln Gly Leu Lys Val Ala Val Ile Val Asn Asp Met Ser Glu
           35                  40                  45
   Val Asn Ile Asp Ala Ala Gln Val Gln Arg Asp Val Ala Leu Tyr Arg
       50                  55                  60
   Gly Gln Asp Glu Leu Ile Glu Met Ser Asn Gly Cys Ile Cys Cys Thr
   65                  70                  75                  80
   Leu Arg Ala Asp Leu Leu Glu Gln Ile Ser Ala Leu Ala Arg Gln Gln
   85 90 95
   Arg Phe Asp Tyr Leu Leu Ile Glu Ser Thr Gly Ile Ser Glu Pro Met
   100 105 110
   Pro Val Ala Glu Thr Phe Ala Phe Leu Asp Ala Asn Gly Phe Ser Leu
   115 120 125
   Ser Glu Leu Ala Arg Leu Asp Thr Leu Val Thr Val Val Asp Ala Ser
   130 135 140
   Gln Phe Met Ala Met Leu Asp Ser Pro Glu Thr Val Ala Arg Ala Asp
   145 150 155 160
   Val Thr Thr Asp Asp Ser Arg Arg Pro Leu Ala Asp Leu Leu Ile Glu
   165 170 175
   Gln Val Glu Tyr Ala Asn Val Ile Leu Val Asn Lys Arg Asp Leu Val
   180 185 190
   Asp Glu Ala Gln Tyr Gln Ala Leu Gln Ala Val Leu Ala Gly Leu Asn
   195 200 205
   Pro Gly Ala Gln Ile Leu Pro Met Val Ala Gly Asn Val Ala Leu Ser
   210 215 220
   Ser Val Leu Gly Thr Gln Leu Phe Asp Leu Pro Ser Leu Ala Ala Ala
   225 230 235 240
   Pro Gly Trp Met Lys Gln Met Asp Ala His Asp Thr Pro Ala Gly Glu
   245 250 255
   Ser Gln Thr Tyr Gly Val Thr Ser Trp Val Tyr Arg Ala Arg Ala Pro
   260 265 270
   Phe His Pro Gln Arg Leu Leu Asp Phe Leu Ala Arg Pro Trp Arg Asp
   275 280 285
   Gly Arg Leu Leu Arg Ser Lys Gly Tyr Phe Trp Leu Ala Ser Arg His
   290 295 300
   Arg Glu Ile Gly Leu Leu Val His Ser Gly Gln Gln Phe Gln Trp Asp
   305 310 315 320
   Tyr Val Gly His Trp Trp Asn Phe Ile Asp Thr Ser Gln Trp Pro Gln
   325 330 335
   Asp Lys Tyr Arg Leu Gln Gly Ile Met Ala Lys Trp Asp Ser Ile Val
   340 345 350
   Gly Asp Cys Arg Gln Glu Leu Lys Ser Leu
   355 360
<210> 11
   <211> 30
   <212> DNA
   <213> Artificial Sequence
<220>
   <223> Primer 1F
<400> 11
   ctccaccata tgagtacagc tacttcaacg 30
<210> 12
   <211> 30
   <212> DNA
   <213> Artificial Sequence
<220>
   <223> Primer 1R
<400> 12
   cttcataagc ttctatctcg gatcaaatgg 30
<210> 13
   <211> 25
   <212> DNA
   <213> Artificial Sequence
<220>
   <223> Primer 2F
<400> 13
   atgacggcaa cttcaacccc tggtg 25
<210> 14
   <211> 20
   <212> DNA
   <213> Artificial Sequence
<220>
   <223> Primer 2R
<400> 14
   tcagctcctg tcggcagtcg 20

## Beschreibung

Die Erfindung betrifft cyanidtolerante Nitrilhydratasen insbesondere aus Pseudomonas putida- oder Pseudomonas marginalis- Stämmen, die eine erhöhte Cyanidtoleranz aufweisen, ihre Verwendung zur Herstellung von Amiden aus Nitrilen in Gegenwart von Cyaniden und für dieses Enzym kodierende Polynukleotidsequenzen.

Die Umsetzung von α-Hydroxynitrilen (Cyanhydrinen) und α-Aminonitrilen zu den entsprechenden Amiden mittels Nitrilhydratasen eröffnet eine neue Synthesevariante zu α-Hydroxysäuren und α-Aminosäuren, da α-Hydroxy- und α-Aminoamide auf einfache Weise verseift werden können (Process and catalysts for the production of methionine. Ponceblanc, Herve; Rossi, Jean-Christophe; Laval, Philip; Gros, Georges. (Rhone-Poulenc Animal Nutrition SA, Fr.), (WO 2001060789). Alternativ können α-Hydroxyamide auch mit Alkali- oder Erdalkalimetallhydroxiden zu den entsprechenden Salzen der Hydroxysäuren umgesetzt werden. Besonders bevorzugt ist hierbei die Umsetzung von 4-Methylthio-a-hydroxybutyramid (MHA-Amid) mit Calciumhydroxid, da Calcium-MHA direkt als alternative Produktform zu Methionin oder MHA als Futtermittelzusatz eingesetzt werden kann.

Allerdings zerfallen α-Hydroxynitrile und α-Aminonitrile leicht zu Aldehyden und Blausäure bzw. Aldehyden, Blausäure und Ammoniak. Die entstehende Blausäure ist ein starker Inhibitor für fast alle bekannten Nitrilhydratasen, mit Ausnahme der Nitrilhydratase aus Rhodococcus equi XL-1, die bei 20 mM Cyanid den bisher geringsten bekannten Aktivitätsverlust zeigt. (Production of amides from nitriles by Rhodococcus equi cells having a cyanide resistant-nitrile hydratase. Nagasawa, Tohru; Matsuyama, Akinobu. (Daicel Chemical Industries, Ltd., Japan), (EP 1 266 962 A).

Die geringe Produktivität von ca. 8 g Amid pro g Biotrockenmasse der Ruhezellen, die lange Reaktionszeit von 43 Stunden und die relativ geringe Produktkonzentration von 75 g/L führen zur Suche nach verbesserten Nitrilhydratasen.

Das Ziel der hier beschriebenen Erfindung ist deshalb einen Biokatalysator zur Verfügung zu stellen, der nicht diesen Einschränkungen unterliegt. Ausserdem ist eine noch höhere Toleranz des Biokatalysators gegenüber Cyanid vorteilhaft, da α-Hydroxynitrile und α-Aminonitrile zur Gewährleistung einer schnellen und vollständigen Umsetzung des Aldehyds bevorzugt mit einem 1-3 %-igen Überschuss an Blausäure hergestellt werden, der zum Teil im Produkt verbleibt. Somit können während der Biotransformation Cyanidkonzentrationen auftreten, die 20 mM übersteigen. Nebenprodukte und Reagentien wie als Hilfsbasen eingesetzte Amine dürfen die Nitrilhydratase-Aktivität ebenfalls nicht inhibieren.

Aufgabe der Erfindung ist es, Nitrilhydratasen bereitzustellen, die gegenüber bei der Umsetzung von Nitrilen zu Amiden in der Reaktionslösung vorhandenen Cyanidionen eine erhöhte Stabilität aufweisen.

Gegenstand der Erfindung sind isolierte Polynukleotide aus Mikroorganismen der Gattung Pseudonomas, die für Polypeptide mit den Aminbsäuresequenzen kodieren, die zu 97 % bis 100 % identisch sind mit den in den Sequenzen SEQ ID NO:2 oder 3 enthaltenden Aminosäuresequenzen, wobei die Polypeptide, enthaltend die Sequenzen SEQ ID NO: 2 oder 3 zusammen jeweils die Aktivität einer cyanidtoleranten Nitrilhydratase besitzen bzw. diese Nitrilhydratase bilden.

Die polynukleotide stammen aus Pseudonomoas marginalis.

Gegenstand der Erfindung sind weiter Polynukleotide, ausgewählt aus der Gruppe
a) Polynukleotide, enthaltend die Nukleotidsequenz SEQ ID NO:1, oder dazu komplementären Nukleotidsequenzen,
b) Polynukleotide, enthaltend,Nukleotidsequenzen, die den Sequenzen aus a) im Rahmen der Degeneriertheit des genetischen Codes entsprechen,
c) Polynukleotide enthaltend Nukleotidsequenzen gemäss a), die funktionsneutrale Sinnmutationen enthalten,
d) Polynukleotide, die mit den komplementären Sequenzen aus a) oder c) unter stringenten Bedingungen hybridisieren,
wobei die Polynukleotide für eine cyanidtolerante Nitrilhydratase kodieren.

Gegenstand der Erfindung sind ebenso die durch diese Polynukleotide kodierten Polypeptide mit den Sequenzen SEQ ID NO:2 oder 3 mit der Aktivität von cyanidtoleranten Nitrilhydratasen aus Mikroorganismen der Gattung Pseudonomas, die sowohl in den Mikroorganismen angereichert oder in isolierter Form vorliegen können. SEQ ID NO:2 kodiert für die alpha-Untereinheits der Nitrilhydratase, SEQ ID NO:3 für die beta-Untereinheit der Nitrilhydratase SEQ ID NO:5 und 10 kodieren für Aktivatorproteine, deren Co-Expression für die Aktivität der Nitrilhydratasen essentiell ist (Nojiri et al., 1999, Journal of Biochemistry, 125:696-704)

Erfindungsgemäß werden Wirtszellen verwendet, die durch die erfindungsgemäßen Polynukleotide transformiert oder transfektiert wurden.

Die Wirtszellen können zu den Eukaryonten oder Prokaryonten zählen, für die ein stabiles Expressionssystem bekannt ist, insbesondere

Als Host-Organismus dienen bevorzugt Mikroorganismen, für Expressionssysteme gibt, wie z. B. Pseudomonas, Pichia, verschiedene Hefen, Saccaromyces, Aspergillus oder der Familie Streptomyces, isbesondere E. coli. Mikroorganismen der Gattung Rhodococcus sind ebenso geeignet.

Vektor DNA kann in eukaryonische oder prokaryonische Zellen durch bekannte Transformations- oder Transfektionstechniken eingeführt werden.

"Transformation", "Transfektion", "Konjugation" i,d "Transduktion" beziehen sich auf nach dem Stand der Techniken bekannten Maßnahmen, um fremde DNA einzuführen.

Gegenstand der Beschreibung sind ebenso Polynukleotide, die im wesentlichen aus einer Polynukleotidsequenz bestehen, die erhältlich sind durch Screening mittels Hybridisierung einer entsprechenden Genbank von Pseudonomas marginalis, die das vollständige Gen oder Teile davon enthält, mit einer Sonde, die die Sequenzen der erfindungsgemäßen Polynukleotide aus der SEQ ID No:1 4 oder 6, 9 oder Fragmente davon enthält und Isolierung der genannten Polynukleotidsequenz.

Polynukleotide, die die Sequenzen gemäß der Beschreibung enthalten, sind als Hybridisierungs-Sonden für RNA, cDNA und DNA geeignet, um Nukleinsäuren beziehungsweise Polynukleotide oder Gene in voller Länge zu isolieren, die für die erfindungsgemäßen Proteine kodieren, oder um solche Nukleinsäuren beziehungsweise Polynukleotide oder Gene zu isolieren, die eine hohe Ähnlichkeit der Sequenzen mit denen der erfindungsgemäßen Gene aufweisen. Sie können ebenso als Sonde auf sogenannte "arrays", "micro arrays" oder "DNA chips" aufgebracht werden, um die entsprechenden Polynukleotide oder hiervon abgeleitete Sequenzen wie z.B. RNA oder cDNA zu detektieren und zu bestimmen.

Polynukleotide, die die Sequenzen gemäß der Beschreibung enthalten, sind weiterhin als Primer geeignet, mit deren Hilfe mit der Polymerase-Kettenreaktion (PCR) DNA von Genen hergestellt werden kann, die für die erfindungsgemäßen Proteine kodieren.

Solche als Sonden oder Primer dienende Oligonukleotide, enthalten mindestens 25 oder 30, bevorzugt mindestens 20, ganz besonders bevorzugt mindestens 15 aufeinanderfolgende Nukleotide. Geeignet sind ebenfalls Oligonukleotide mit einer Länge von mindestens 40 oder 50 Nukleotiden. Gegebenenfalls sind auch Oligonukleotide mit einer Länge von mindestens 100, 150, 200, 250 oder 300 Nukleotiden geeignet.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Die Polynukleotide gemäß Erfindung schließen Polynukleotide gemäß SEQ ID No:1 und auch solche ein, die zu wenigstens 90 %, 93 %, 95 %, 97 % oder 99% identisch sind mit den Polynukleotiden gemäß SEQ ID NO:1.

Unter polypeptiden" versteht man Peptide oder Proteine, die zwei oder mehr über Peptidbindungen verbundene Aminosäuren enthalten.

Die Polypeptide gemäß Erfindung schließen Polypeptide gemäß den Sequenzen SEQ ID NO:2, 3, und auch solche ein, die zu wenigstens 97% oder 99% identisch sind mit den Polypeptiden gemäß den Sequenzen SEQ ID NO:2, 3.

Die aus der gewünschten Genbank erhaltenen DNA-Sequenzen können dann mit bekannten Algorithmen bzw. Sequenzanalyse-Programmen wie z.B. dem von Staden (Nucleic Acids Research 14, 217-232(1986)), dem von Marck (Nucleic Acids Research 16, 1829-1836 (1988)) oder dem GCG-Programm von Butler (Methods of Biochemical Analysis 39, 74-97 (1998)) untersucht werden.

Kodierende DNA-Sequenzen, die sich der in aus SEQ ID No. 1, enthaltenen Sequenz durch die Degeneriertheit des genetischen Kodes ergibt, ist ebenfalls Bestandteil der Erfindung. In gleicher Weise sind DNA-Sequenzen, die mit diesen Sequenzen oder Teilen von davon unter stringenten Bedingungen hybridisieren, Bestandteil der Erfindung. In der Fachwelt sind weiterhin konservative Aminosäureaustausche wie z.B. Austausch von Glycin gegen Alanin oder von Asparaginsäure gegen Glutaminsäure in Proteinen als "Sinnmutationen" ("sense mutations") bekannt, die zu keiner grundsätzlichen Veränderung der Aktivität des Proteins führen, d.h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie.

Schließlich sind DNA-Sequenzen Bestandteil der Beschreibung, die durch die Polymerase-Kettenreaktion (PCR) unter Verwendung von Primern hergestellt werden, die sich aus SEQ ID No: 1, 4, 6, 9 ergeben. Derartige Oligonukleotide haben typischerweise eine Länge von mindestens 15 aufeinanderfolgenden Nukleotiden, insbesondere von 20, 30 oder 40.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 90% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird vorzugsweise bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein 5x SSC-Puffer bei einer Temperatur.von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca 50°C- 68°C eingestellt wird. Es ist erfindungsgemäß die Salzkonzentration bis auf 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 90% bis 95% Identität zur Sequenz der eingesetzten Sonde besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Anleitungen zur Amplifikation von DNA-Sequenzen mit Hilfe der Polymerase-Kettenreaktion (PCR) findet der Fachmann unter anderem im Handbuch von Gait: Oligonukleotide synthesis: A Practical Approach (IRL Press, Oxford, UK, 1984) und bei Newton und Graham: PCR (Spektrum Akademischer Verlag, Heidelberg, Deutschland, 1994).

Im allgemeinen geht man so vor, dass man ein gut exprimierbares Gen in einen Vektor mit niedriger Kopienzahl, Gene mit schwächerer Expressionsleistung auf einem Vektor mit höherer Kopienzahl und/oder starkem Promotor kloniert. Die Wirtszellen sind mit diesen Vektoren in der Weise transformiert, dass sie im Vergleich zum Startorganismus mindestens jeweils eine zusätzliche Kopie der für die Bildung von Nitrilhydratase kodierenden Nukleotidsequenzen enthalten.

Die so hergestellten transformierten oder rekombinanten Mikroorganismen insbesondere der Gattung Pseudonomas sind ebenfalls Teil der Erfindung.

Es wurde gefunden, dass die Verstärkung der für die erfindungsgemäße Nitrylhydratase und das Helferprotein P47K kodierenden Gene in Mikroorganismen zu einer erhöhten Produktion der Nitrilhydratase oder auch zu einer erhöhten Aktivität der Nitrilhydratase führen.

Der Begriff "verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert, im Vergleich zum nicht rekombinierten Startorganismus.

Zur Erzielung einer überexpression kann die Promotor- und Regluationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen Aminosäure-Produktion zu steigern. Durch Maßnahmen zur Verlängerung der Lebensdauer der m-RNA wird ebenfalls die Expression verbessert.

Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Gegenstand der Erfindung sind ebenso
1) ein Verfahren zur enzymatischen Herstellung von Amiden aus Nitrilen, das folgende Schritte aufweist:
   a) Umsetzung einer (eine) Nitrilgruppe(n) enthaltenden Verbindung mit einem mikrobiellen Enzym, das Nitrilhydratase-Aktivität aufweist und
   b) Abtrennung des gebildeten Amids, wobei man
   c) für die Umsetzung des Nitrils zum Amid eine erfindungsgemäße Nitrilhydratase einsetzt. Deren Restaktivität beträgt nach der Umsetzung von Methacrylnitril in Gegenwart von 20 mM (mM = mmol/1) Cyanidionen bei 20°C nach 30 min. mindestens 90 % der Restaktivität desselben Enzyms, wenn dieses das unter ansonsten denselben Bedingungen in Abwesenheit von Cyanidionen für die Umsetzung eingesetzt wurde.
2) ein Verfahren gemäß 1), dadurch gekennzeichnet, dass die Restaktivität nach der Umsetzung in Gegenwart von 50 mM Cyanidionen mindestens 60 % beträgt,
3) ein Verfahren gemäß 1) oder 2), dadurch gekennzeichnet, dass man das Enzym produzierende und enthaltende Mikroorganismen oder deren Lysat einsetzt.
4) ein Verfahren gemäß 3), dadurch gekennzeichnet, dass man ruhende Zelle des Mikroorganismus einsetzt,
5) ein Verfahren gemäß 1) oder 2), dadurch gekennzeichnet, dass man das gereinigte Enzym einsetzt,
6) ein Verfahren gemäß 1) bis 5), dadurch gekennzeichnet, dass das Enzym aus Mikroorganismen der Spezies Pseudonomas putida oder Pseudonomas marginalis stammt.
7) ein Verfahren gemäß 6, dadurch gekennzeichnet, dass das Enzym aus Mikroorganismen der Gattung Pseudomonas stammt, hinterlegt unter den Nummern DSM 16275 und DSM 16276, und die Aminosäuresequenzen mit den Sequenzen SEQ ID NO:2, 3, aufweisen,
8) ein Verfahren gemäß einem oder mehreren der Punkte 1) bis 7), dadurch gekennzeichnet, dass man Verbindungen der allgemeinen Formeln

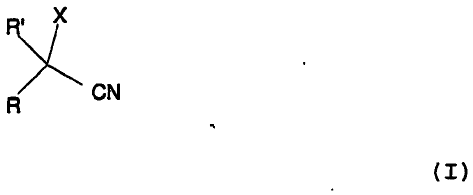

   R"-CN (II)

   in der bedeuten:
   - X:: OH, H, Alkyl mit 1 bis 4 C-Atomen, NH₂
   - R:: H, gesättigter Alkylrest mit 1 bis 12 C-Atomen, verzweigt oder unverzweigt, gegebenenfalls NH₂-substituiert
   ungesättigte Alkylreste mit einer Doppelbindung und 1 bis 12 C-Atomen, verzweigt oder unverzweigt, Cycloalkylgruppen mit 3 bis 6 C-Atomen, mit Alkylthiogruppen substituierte Alkylenreste, wobei Alkyl hier einem C₁ bis C₃-Rest

   und Alkylen einem zweiwertigen C₃ bis C₈-Rest entspricht,
   - R':: H, wenn R kein H bedeutet, Alkyl mit 1 bis 3 C-Atomen,
   - R":: ein- oder zweikerniger ungesättigter Ring, mit 6 bis 12 C-Atomen, gegebenenfalls substituiert mit einer oder zwei Alkylgruppen (C₁ - C₃), Cl, BR,F substituiert,
   einwertiger Alkylnitrilrest mit 1 bis 6 C-Atomen zu den entsprechenden Amiden umsetzt,
9) ein Verfahren gemäß 8), dadurch gekennzeichnet, dass man eine Verbindung der allgemeinen Formel (I) in Gegenwart von Blausäure oder einem Salz der Blausäure umsetzt,
10) ein Verfahren gemäß 9), dadurch gekennzeichnet, dass man die Umsetzung in Gegenwart von 0,1 mol% Cyanid bis 3 mol% Cyanid bezogen auf das eingesetzte Nitril durchführt, bevorzugt > 2 bis 3 mol%. Dies entspricht bei 1 mol Endkonzentration bei 3 mol% 30 mMol Cyanid,
11) ein Verfahren gemäß einem oder mehreren der Punkte 1) bis 10), dadurch gekennzeichnet; dass man als Nitril Methioninnitril einsetzt,
12) ein Verfahren gemäß einem oder mehreren der Punkte 1) bis 10), dadurch gekennzeichnet, dass man als Nitril 2-Hydroxy-4-methylthiobutyronitril einsetzt.
   Bevorzugt setzt man ein Reaktionsgemisch ein, wie man es erhält , wenn man Blausäure, 3-Methylthiopropionaldehyd in Gegenwart einer Hilfsbase wie z.B. Triethylamin nach dem Stand der Technik umsetzt.
   Es kann vorteilhaft ohne Aufreinigung eingesetzt werden.
   Dies weist auf die zusätzliche Stabilität der erfindungsgemäßen Enzyme gegenüber Aldehyden und Aminen hin.
13) Ein Verfahren, bei dem man als Vorstufe für Methacrylamid 2-Hydroxy-2-methylpropionitril, einsetzt.
14) Die Erfindung ist ebenso ausgerichtet auf isolierte und gereinigte Mikroorganismen der Gattung Pseudomonas, hinterlegt unter den Nummern DSM 16275 (MA32, Pseudomonas marginalis) und DSM 16276 (MA113, Pseudomonas putida), und
15) Cyanidtolerante Nitrilhydratasen, isoliert aus den Stämmen der Gattung Pseudomonas, insbesondere aus den unter den Nummern DSM 16275 und DSM 16276 hinterlegten Stämmen von Pseudonomas putida und Pseudonomas marginalis.

Die Hinterlegung erfolgte am 09.03.2004 bei der DSMZ, Deutsche Sammlung für Mikroorganismen und Zellkulturen in Braunschweig, nach dem Budapester Vertrag.

Diese Stämme sind besonders geeignet, die erfindungsgemäßen Enzyme zu produzieren.

"Isolierte und gereinigte Mikroorganismen" betrifft Mikroorganismen, die in einer höheren Konzentration als natürlich zu finden vorliegen.

Gegenstand der Beschreibung ist ebenso ein Verfahren zur Herstellung der oben beschriebenen cyanidtoleranten Nitrilhydratase, bei dem man
a) einen diese Nitrilhydratase produzierenden Mikroorganismus, insbesondere der Gattung Pseudomonas marginalis oder Pseudomonas putida, unter Bedingungen fermentiert, bei denen sich das Enzym in dem Mikroorganismus bildet, und
b) frühestens nach dem Durchlaufen der logarithmischen Wachstumsphase die Zellen erntet.

Anschließend setzt man
a) entweder den das Enzym enthaltenden Mikroorganismus als in Form von ruhenden Zellen, gegebenenfalls nach der Erhöhung der Permeabilität der Zellmembran oder
b) das Lysat der Zellen oder
c) das aus den Zellen des Mikroorganismus mit bekannten Maßnahmen isolierte Enzym
zur erfindungsgemäßen umwandlung von Nitrilen in Amide ein.

Bei der Nitrilhydratase kann es sich sowohl um ein mit nicht rekombinanten Mikroorganismen erzeugtes als auch um ein rekombinant erzeugtes Enzym handeln.

Gegenstand der Beschreibung sind weiterhin Verfahren zur rekombinanten Herstellung der erfindungsgemäßen Polypeptide, wobei man einen diese Polypeptide produzierenden Mikroorganismus kultiviert, gegebenenfalls die Expression der zugehörigen Polynukleotide induziert und die Enzyme gegebenenfalls aus der Kultur isoliert.

Es handelt sich im allgemeinen um ein Verfahren, bei dem man
a) Mikroorganismen insbesondere der Gattungen Pseudonomas marginalis oder Pseudonomas putida fermentiert, in denen man isolierte Polynukleotide aus Mikroorganismus der Familie Pseudonomas, die für Polypeptide mit den Aminosäuresequenzen kodieren, die zu 90 bis 100 % identisch sind mit den Sequenzen in den SEQ ID NO:2, 3 und 5 oder 7, 8 und 10 enthaltenden Aminosäuresequenzen, wobei die Polypeptide jeweils gemeinsam die Aktivität einer cyanidtoleranten Nitrilhydratase besitzen, verstärkt, insbesondere rekombinant überexprimiert,
b) aus diesen Mikroorganismen das Enzym mit Nitrilhydrataseaktivität gegebenenfalls isoliert oder eine dieses Enzym enthaltende Proteinfraktion herstellt, und
c) die Mikroorganismus gemäss a) oder das Enzym gemäss oder die dieses enthaltende Fraktion b) in ein Medium überführt, das ein Nitrilgruppen-haltige Verbindung der allgmeinen Formeln (I) und (II) enthält.

Das zur Fermentation verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Lactose, Fructose, Maltose, Melasse, Stärke und Cellulose, Öle und Fette wie z.B. Sojaöl, Sonnenblumenöl, Erdnußöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können vorteilhaft organische Nitrile oder Säureamide wie Acetonitril, Acetamid, Methacrylnitrile, Methacrylamid, Isobutyronitril, Isobutyramid oder Harnstoff auch in Kombination mit anderen Stickstoffhaltigen Verbindungen wie Peptone, Hefeextrakt, Fleischextrakt, Malzextrakt, Maisquellwasser, Sojabohnenmehl und oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Um aerobe Bedingungen aufrechtzuerhalten, werden Sauerstoff oder Sauerstoff haltige Gasmischungen wie z.B. Luft in die Kultur eingetragen. Die Temperatur der Kultur liegt normalerweise bei 10°C bis 40°C und vorzugsweise bei 10°C bis 30°C. Die Kultur wird solange fortgesetzt, bis sie die logarithmische Wachstumsphase durchschritten hat. Dieses Ziel wird normalerweise innerhalb von 10 Stunden bis 70 Stunden erreicht. Im Anschluss daran werden die Zellen bevorzugt geerntet, gewaschen und in einem Puffer als Suspension bei einem pH-Wert von 6-9, insbesondere von 6,8 bis 7,9 aufgenommen. Die Zellkonzentration beläuft sich auf 1-25%, insbesondere 1,5 bis 15% (Feuchtgewicht/v). Die Permeabilität kann mit physikalischen oder chemischen Methoden so, z. B. mit Toluol wie bei Wilms et al., J. Biotechnol., Vol. 86 (2001), 19-30 beschrieben, erhöht werden, dass das umzuwandelnde Nitril die zellwand durchdringen und das Amid austreten kann.

Folgende Nitrile werden bevorzugt umgesetzt:
gesättigte Mononitrile:
   Acetonitril, Propionitril, Butyronitril, Isobutyronitril, Valeronitril, Isovaleronitril, Capronitril
gesättigte Dinitrile:
   Malonitril, Succinonitril, Glutaronitril, Adiponitril
aromatische unsubstituierte und substituierte Mono- und Dinitrile:
   Benzonitril, 2,6-Difluorbenzonitril, Phthalonitril, Isophthalonitril, Terephthalonitril,
α-Aminonitrile:
   α-Aminopropionitril, α-Aminomethylthiobutyronitril, α-Aminobutyronitril, Aminoacetonitril, alle von natürlichen Aminosäuren abgeleitete Nitrile, α-Amino-3,3-dimethylpropionitril α-Amino-2,3-dimethylpropionitril
Nitrile mit Carboxyl-Gruppen:
   Cyanessigsäure
β-Aminonitrile:
   Amino-3-propionitril
ungesättigte Nitrile:
   Acrylnitril, Methacrylonitril, Allylcyanid, Crotononitril
α-Hydroxynitrile:
   α-Hydroxy-n-propionitril, α-Hydroxy-n-butyronitril, α-Hydroxy-isobutyronitril, α-Hydroxy-n-hexanonitril, α-Hydroxy-n-heptanonitril, α-hydroxy-n-octanonitril, α,γ-Dihydroxy-β, β-dimethylbutyronitril, Acroleincyanohydrin, Methacrylaldehyd cyanohydrin, 3-Chlorolactonitril, 4-Methylthio-α-hydroxybutyronitril und α-Hydroxyphenylpropionitril.

Die Konzentration der umzusetzenden Nitrile in der Reaktionslösung ist nicht auf bestimmte Bereiche begrenzt.

Um eine Inhibierung der Enzymaktivität durch das Substrat zu vermeiden, hält man die Konzentration des Nitrils im allgemeinen auf 0,02 bis 10 w/w%, insbesondere 0,1 bis 2 w/w%, bezogen auf die Menge des Biokatalysators als getrocknete Zellmasse. Das Substrat kann zu Beginn der Umsetzung insgesamt oder im Verlauf der Umsetzung kontinuierlich oder diskontinuierlich zugesetzt werden.

Die Bestimmumng des Trockengewichts erfolgt mit dem Moisture Analyser MA 45 (Sartorius).

Wenn die Löslichkeit der Nitrilverbindung in dem wässrigen Reaktionssystem zu gering ist, kann ein Lösungsvermittler zugesetzt werden.

Die Reaktion kann aber alternativ auch in einem Zweiphasensystem Wasser/organische Lösungsmittel durchgeführt werden.

Bei der Verwendung von Zellen des Mikroorganismus als enzymatisch aktivem Material, ist die Menge der eingesetzten Zellen im Verhältnis zur Substratmenge bevorzugt 0,02 bis 10 w/w% als getrocknete Zellmasse.

Es ist auch möglich, das isolierte Enzym nach allgemein bekannten Techniken zu immobilisieren und in dieser Form dann einzusetzen.

Die Reaktion wird im allgemeinen bei Temperaturen von -5°C bis 50°C, insbesondere 0°C bis 30°C, und einer Zeitdauer von 0,1 bis 100 Stunden durchgeführt.

Der einzuhaltende pH-Wert des Reaktionsgemisches ist so lange nicht auf bestimmte Werte begrenzt, wie die enzymatische Aktivität nicht beeinträchtigt wird. Nach der Umsetzung kann das gebildete Amid aus der Reaktionslösung wie bekannt abgetrennt und gereinigt werden.

Gegenstand der Erfindung ist ebenfalls ein Verfahren, bei dem man das Amid bzw. die das Amid enthaltende Lösung zum Beispiel von den Zellen der Biomasse abtrennt und das Amid entweder zu der entsprechenden Säure verseift oder unter Zusatz von Alkali- oder Erdalkalimetallyhydroxiden zu den entsprechenden Salzen der Säuren umsetzt. Bevorzugt wird MHA-Amid mit Calziumhydroxid verseift und das entsprechende Calziumsalz isoliert.

### Beispiele

### Beispiel 1

### Anzuchtbedingungen

Die Vorkulturen wurden innerhalb von 24 h unter Schütteln bei 30°C in einem Volumen von 5 ml in Glasröhrchen angezogen. Mit 1 ml der Vorkultur wurden 100 ml der Hautkultur angeimpft und 42 h bei 25°C in einem Erlenmeyerkolben mit einem Gesamtvolumen von 1000 ml geschüttelt.

| Medium für die Vorkultur (pH 7,0) | |
|---|---|
| K₂HPO₄ | **7 g** |
| KH₂PO₄ | **3 g** |
| Na-citrat | **0,5 g** |
| Glycerin | **2 g** |
| FeSO4 * 7 H₂O | **0,004 g** |
| MgSO4 * 7 H₂O | **0.1 g** |
| Acetamid | **2 g** |
| Spurensalzlösung | **0,1 ml** |
| Demineralisiertes Wasser | **Ad. 1000 ml** |

| Medium für die Hauptkultur (pH 7,0) | |
|---|---|
| K₂HPO₄ | **7 g** |
| KH₂PO₄ | **3 g** |
| Natriumcitrat | **0,5 g** |
| Glycerin | **2 g** |
| FeSO4 * 7 H₂O | **0,004 g** |
| MgSO4 * 7 H₂O | **0.1 g** |
| Acetamid | **10 g** |
| Spurensalzlösung | **0,1 ml** |
| Demineralisiertes Wasser | **Ad. 1000 ml** |

| Spurensalzlösung | |
|---|---|
| EDTA, Na₂ * 2 H₂O | **158 mg** |
| Na₂MoO₄ * 2 H₂O | **4,7 mg** |
| ZnSO₄ * 7 H₂O | **70 mg** |
| MnSO₄ * 4 H₂O | **18 mg** |
| FeSO₄ * 7 H₂O | **16 mg** |
| CuSO₄ * 5 H₂O | **4,7 mg** |
| CoSO₄ * 6 H₂O | **5,2 mg** |
| Demineralisiertes Wasser | **Ad. 1000 ml** |

### Beispiel 2

### Isolierung und Identifizierung der Mikroorganismen

Die beiden Stämme MA32 und MA113 wurden durch Bestimmung der Nitrilhydratase-Aktivität der Ruhezellen in Gegenwart von 2 mM Kaliumcyanid selektiert.

### Eigenschaften von MA32:

| Zellform | | Stäbchen |
|---|---|---|
| Breite | | 0,6 - 0,8 µm |
| Länge | | 1,5 - 3,0 µm |
| Beweglichkeit | | + |
| Geißeln | | polar > 1 |
| | | |
| Gram-Reaktion | | - |
| Lyse durch 3% KOH | | + |
| Aminopeptidase (Cerny) | | + |
| Oxidase | | + |
| Katalase | | + |
| | | |
| Wachstum bei 41°C | | - |
| | | |
| Substratverwertung | | |
| | Adipat | - |
| | Citrat | + |
| | Malat | + |
| | Phenylacetat | - |
| | D-Glucose | + |
| | Maltose | - |
| | Mannitol | + |
| | Arabinose | + |
| | Mannose | + |
| | Trehalose | + |
| | Sorbitol | + |
| | Erythrol | + |
| | Citraconat | + |
| | Inositol | + |
| | | |
| ADH | | + |
| Urease | | - |
| | | |
| Hydrolyse von Gelatine | | + |
| Hydrolyse von Esculin | | + |
| | | |
| Levan aus Saccharose | | + |
| | | |
| Denitrification | | + |
| | | |
| Lecithinase | | + |
| | | |
| Fluoreszens | | + |
| Pyocyanin | | - |

Das Profil der zellulären Fettsäuren ist typisch für die Gruppe I der Pseudomonaden

Die Analyse eines 484 bp langen Abschnitts der 16S rRNA ergab eine 100%-ige Übereinstimmung mit der Sequenz von *Pseudomonas marginalis*

Unter Berücksichtigung aller Daten konnte MA32 als *Pseudomonas marginalis* identifiziert werden.

### Eigenschaften von MA113:

| | | |
|---|---|---|
| Zellform | | Stäbchen |
| Breite | | 0,6 - 0,8 µm |
| Länge | | 1,5 - 3,0 µm |
| | | |
| Beweglichkeit | | + |
| Geißeln | | polar > 1 |
| | | |
| Gram-Reaktion | | - |
| Lyse durch 3% KOH | | + |
| Aminopeptidase (Cerny) | | + |
| Oxidase | | + |
| Katalase | | + |
| | | |
| Wachstum bei 41°C | | - |
| | | |
| Substratverwertung | | |
| | Adipat | - |
| | Citrat | + |
| | Malat | + |
| | Phenylacetat | + |
| | D-Glucose | + |
| | Maltose | - |
| | Mannitol | - |
| | Arabinose | |
| | Mannose | - |
| | Trehalose | - |
| | Inositol | - |
| | β-Alanin | + |
| | α-Ketoglutarat | + |
| | Benzylamin | + |
| | Hippurat | + |
| | Azelat | + |
| | D-Mandelat | + |
| | | |
| ADH | | + |
| Urease | | - |
| | | |
| Hydrolyse von Gelatine - | | |
| Hydrolyse von Esculin | | - |
| | | |
| Levan aus Saccharose | | - |
| Denitrification | | - |
| | | |
| Lecithinase | | - |
| | | |
| Fluoreszens | | + |
| Pyocyanin | | - |

Das Profil der zellulären Fettsäuren ist typisch für die Gruppe I der Pseudomonaden

Die Analyse eines 476 bp langen Abschnitts der 16S rRNA ergab eine 100%-ige Übereinstimmung mit der Sequenz von *Pseudomonas putida*

Unter Berücksichtigung aller Daten konnte MA113 als *Pseudomonas putida* identifiziert werden.

### Beispiel 3

### Bestimmung der enzymatischen Aktivität

Die Zellen wurden wie in Beispiel 1 beschrieben angezogen, durch zentrifugation vom Kulturmedium abgetrennt und im Standardpuffer (50 mM Kaliumphosphatpuffer pH 7,5) resuspendiert. 50 µl dieser Zellsuspension wurden zu 700 µl des Standardpuffers gegeben und zum Starten der Reaktion mit 250 µl einer 200 mM Lösung des Nitrils in Standardpuffer versetzt. Die Konzentration der Zellen in der Zellsuspension war hierbei so bemessen, daß das Nitril nach 10 min bei 20°C zu 5-30 % umgesetzt war. Nach 10 min bei 20°C wurde die Reaktion durch Zugabe von 20 µl halbkonzentrierter Phosphorsäure abgestoppt und die Zellen wurden durch Zentrifugation abgetrennt.

| HPLC-Analytik | |
|---|---|
| Säule | **Intersil ODS-3V (GL Sciences Inc.)** |
| Mobile Phase | **Gemisch aus 10 mM Kaliumphosphatpuffer pH 2,3 und Acetonitril im Verhältnis 85:15 für Methioninnitril, MHA-Nitil und Acetoncyanhydrin bzw. 99:1 für alle anderen Substrate** |
| Flußrate | **1 ml/min** |
| Detektion | **UV bei 200 nm** |

Die Aktivität von einem U ist definiert als die Menge an Enzym, die 1 µmol Methacrylnitril in einer Minute zum Amid umsetzt. Entstand neben dem Amid auch die Säure, wurde ein U definiert als die Menge an Enzym, die 1 µmol Methacrylnitril in einer Minute zu Amid und Säure umsetzt.

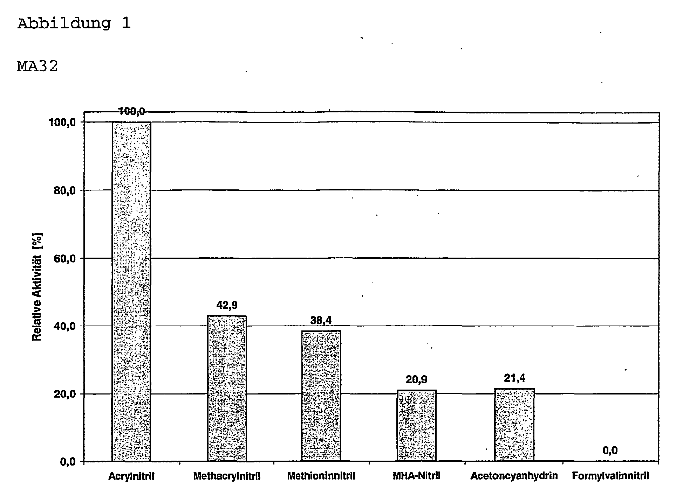

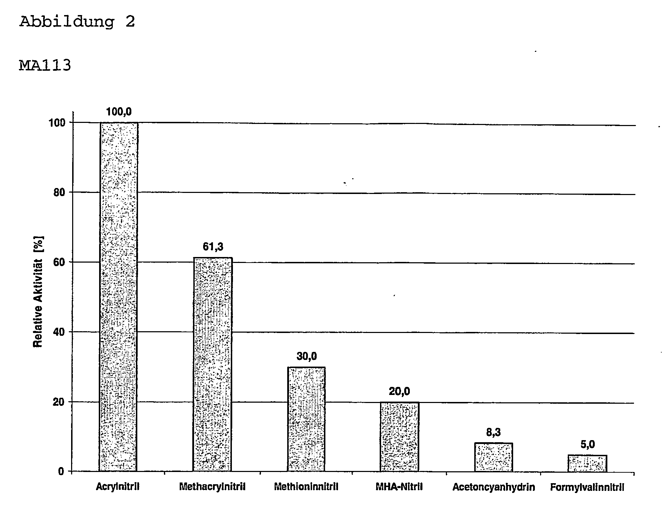

In Abbildung 1 und in Abbildung 2 werden die relative Aktivitäten der Stämme MA32 und MA113 dargestellt.

### Beispiel 4

### Einfluß von Cyanid auf die Aktivität der Nitrilhydratase

50 µl einer analog zu Beispiel 3 hergestellten Zellsuspension wurden zu 700 µl des Standardpuffers gegeben, der 0; 21,4; 53,6 und 107,1 mM Kaliumcyanid enthielt (Endkonzentration 0, 20, 50, 100 mM Cyanid. Zum starten der Reaktion wurden 200 µl einer 200 mM Lösung des Nitrils im Standardpuffer zugesetzt, der jeweils die selbe Cyanidkonzentration aufwies wie die übrige Reaktionslösung. Die Konzentration der Zellen in der Zellsuspension war hierbei so bemessen, daß das Nitril im Ansatz ohne Cyanid nach 10 min bei 20°C zu 16 % umgesetzt war. Nach 10 min bei 20°C wurde die Reaktion durch Zugabe von 20 µl halbkonzentrierter Phosphorsäure abgestoppt und der Umsatz wurde analog zu Beispiel 2 bestimmt.

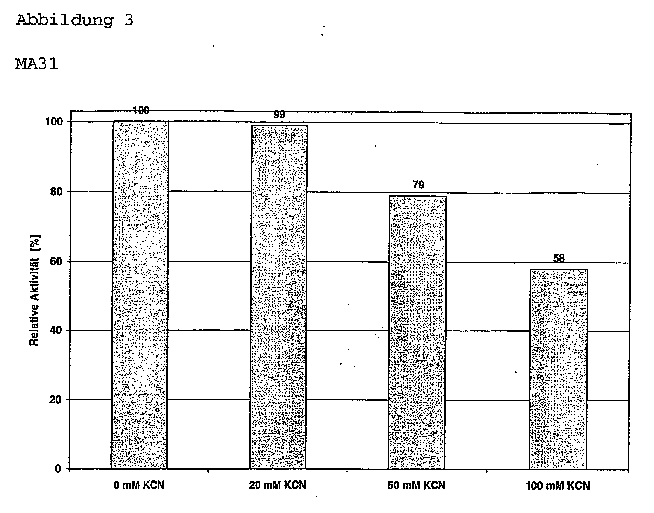

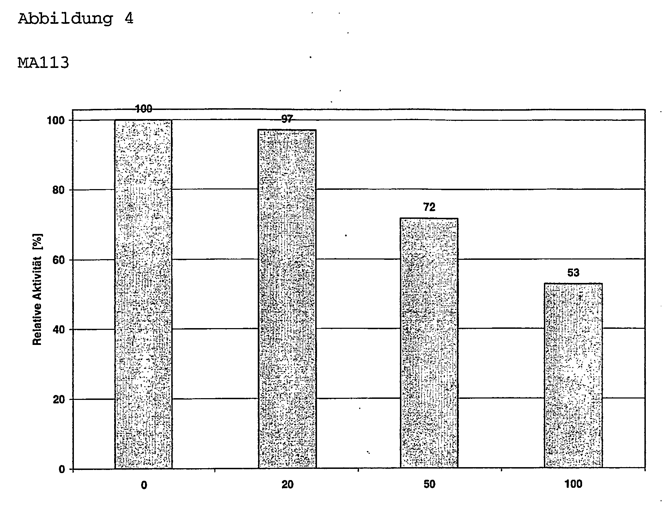

In Abbildung 3 und in Abbildung 4 werden die relativen Aktivitäten für die Umsetzung von Methacrylnitril in Abhängigkeit von der Cyanidkonzentration wiedergegeben.

### Beispiel 5

### Umsetzung von Acetoncyanhydrin mit Pseudomonas marginalis MA32 Ruhezellen

*Pseudomonas marginalis* MA32 Zellen wurden wie in Beispiel 1 beschrieben angezogen und abzentrifugiert. Eine solche Menge der Zellen, die 1,16 g Biotrockenmasse enthielt, wurde mit 50 mM Kaliumphosphatpuffer pH 8,0 auf ein Endvolumen von 50 ml verdünnt. Zusätzlich wurden dem Reaktionsgemisch 0,02 mM 2-Methyl-1-propanboronsäure zugesetzt. Frisch destilliertes Acetoncyanhydrin wurde bei 4°C unter heftigem Rühren kontinuierlich mit einer solchen Rate zugegeben, daß die Konzentration während der Reaktion 5 g/L zu keinem Zeitpunkt überschritt. Der pH wurde konstant bei 7,5 gehalten. Die Reaktionsverfolgung wurde mittels HPLC wie in Beispiel 3 beschrieben durchgeführt. Nach 140 min waren 10,0 g des Nitrils vollständig zu 10,7 g Amid und 1,4 g Säure umgesetzt worden.

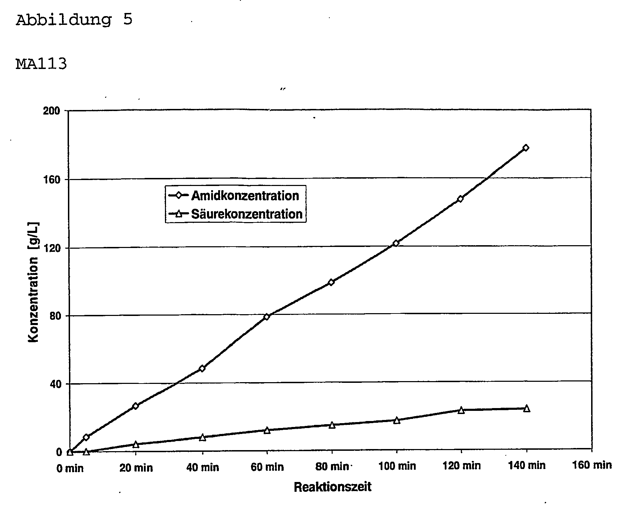

In Abbildung 5 wird der mit dem Stamm MA113 erzielte zeitliche Reaktionsablauf dargestellt.

### Beispiel 6

### Umsetzung von rohem MHA-Nitril mit Pseudomonas marginalis MA32 Ruhezellen

*Pseudomonas marginalis* MA32 Zellen wurden wie in Beispiel 1 beschrieben angezogen und abzentrifugiert. Eine solche Menge der Zellen, die 0,34 g Biotrockenmasse enthielt, wurde mit 50 mM Kaliumphosphatpuffer pH 8,0 auf ein Endvolumen von 70 ml verdünnt. Zusätzlich wurden dem Reaktionsgemisch 0,02 mM 2-Methyl-1-propanboronsäure zugesetzt. Das rohe MHA-Nitril wurde bei 4°C unter heftigem Rühren kontinuierlich mit einer solchen Rate zugegeben, daß die Konzentration während der Reaktion 10 g/L zu keinem Zeitpunkt überschritt. Der pH wurde konstant bei 8,0 gehalten. Die Reaktionsverfolgung wurde mittels HPLC wie in Beispiel 2 beschrieben durchgeführt. Nach 510 min waren 10,05 g des Nitrils vollständig zu 11,13 g Amid und 0,31 g Säure umgesetzt worden. Das entspricht einer Endkonzentration von 139 g Amid pro Liter.

Das MHA-Nitril war direkt aus 3-Methylthiopropionaldehyd und einem leichten Überschuss an Blausäure hergestellt worden. Eine 50 mM Lösung dieses MHA-Nitrils in Wasser enthielt 0,5 mM Cyanid (Spektroquant^{®}, Merck).

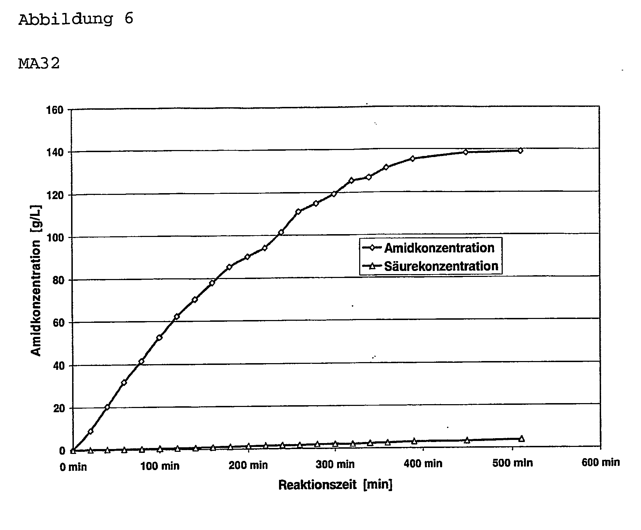

In Abbildung 6 wird der mit dem Stamm MA32 erzielte zeitliche Reaktionsablauf dargestellt.

### Beispiel 7

### Klonierung des Nitrilhydratase-Gen-clusters aus Pseudomonas marginalis MA 32 und Konstruktion eines Expressionsvektors

Der Gen-Cluster der Nitrilhydratase enthaltend eine α-Untereinheit, β-Untereinheit und einem Nitrilhydratase-Aktivatorprotein, dessen Co-Expression für die Aktivität der Nitrilhydratase essentiell ist (Nojiri et al., 1999, Journal of Biochemistry, 125:696-704), wurde mit den Primern 1F und 1R per PCR amplifiziert, die Schnittstellen für die Restriktionsenzyme NdeI und HindIII einfügten. Das so erhaltene PCR-Produkt wurde in einen mit NdeI und HindIII geschnittene Vekor ligiert, bei dem die eingefügten Gene unter der Kontrolle des Rhamnose-Promotors stehen. Der so entstandene Expressionsvektor heißt pKE31.

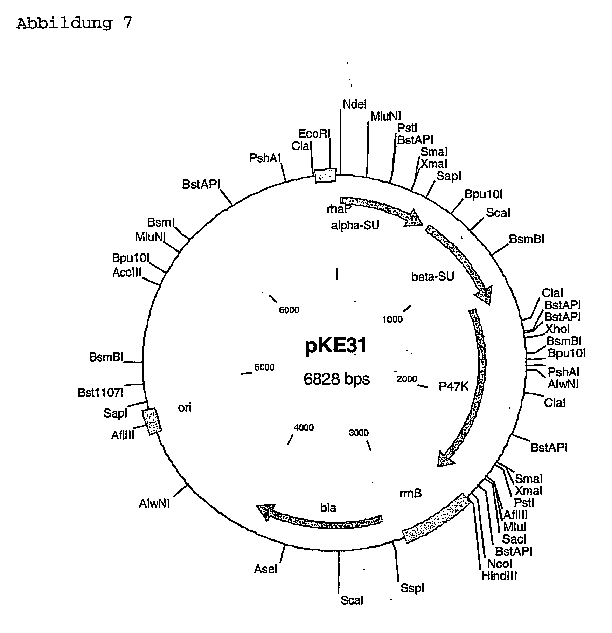

Die Restriktionskarte findet sich in Abbildung 7, die Sequenz unter SEQ ID NO:1.

Das Expressionsplasmid wurde in den Stamm E. coli DSM 14459 transformiert, der bei der Deutschen Sammlung von Mikroorganismen und Zellkulturen GmbH (DSMZ) am 22.08.2001 hinterlegt worden ist.

**Primer:**

| | |
|---|---|
| 1F | 5'-CTC CAC CAT ATG AGT ACA GCT ACT TCA ACG -3' |
| 1R | 5'-CTT CAT AAG CTT CTA TCT CGG ATC AAA TGG-3' |

| | |
|---|---|
| 1F: SEQ ID NO:11 1R: SEQ ID NO:12 | |

Die Gene befinden sich auf den Abschnitten von SEQ ID NO:1:

| | | |
|---|---|---|
| Gen der α-Untereinheit: | nt | 25-609 |
| Gen der β-Untereinheit: | nt | 650-1312 |
| Gen des Aktivatorproteins: | nt | 1309-2577 |

### Beispiel 8

### Klonierung des Nitrilhydratase-Gen-clusters aus Pseudomonas putida MA113

Der Gen-Cluster der Nitrilhydratase bestehend aus α-Untereinheit, β-Untereinheit und einem Nitrilhydratase-Aktivatorprotein, dessen Co-Expression für die Aktivität der Nitrilhydratase essentiell ist (Nojiri et al., 1999, Journal of Biochemistry, 125:696-704), wurde mit den Primern 1F und 1R per PCR amplifiziert.

Die Sequenz findet sich unter SEQ ID NO:6.

**Primer:**

| | |
|---|---|
| 2F | 5'-ATG ACG GCA ACT TCA ACC CCT GGT G-3' |
| 2R | 5'-TCA GCT CCT GTC GGC AGT CG-3' |

| | |
|---|---|
| 2F:SEQ ID NO:13 2R:SEQ ID NO:14 | |

Die Gene befinden sich auf den Abschnitten von SEQ ID NO:5:

| | | |
|---|---|---|
| Gen der α-Untereinheit: | nt | 1-582 |
| Gen der β-Untereinheit: | nt | 624-1286 |
| Gen des Aktivatorproteins: | nt | 1283-2360 |

### Beispiel 9

### Heterologe Expression der Nitrilhydratasen aus Pseudomonas marginalis MA 32 in E. coli DSM 14459

E. coli DSM 14459 wurde im Zusammenhang mit der DE 101 55 928 hinterlegt.

Die mit pKE31 transformierten Zellen wurden in LB-Medium (LB Bouillon nach Miller, VWR), das 2 mM Eisen(III)-Citrat und 100 µg/ml Ampicillin enthielt, unter Schütteln bei 37°C angezogen. Nach 12 - 16 Stunden wurde eine solche Menge der Vorkultur in eine Hauptkultur überimpft, dass diese eine OD600 von 0,1 aufwies. Das Kulturmedium der Hauptkultur entsprach dem der Vorkultur, enthielt aber zusätzlich 2 g/L L-Rhamnose. Die Ernte der Zellen erfolgte nach 22 stündiger Kultivierung bei 30°C.

### Beispiel 10

### Bestimmung der enzymatischen Aktivitäten

Die Anzucht der Zellen und die Bestimmung der Aktivität wurden wie in Beispiel 9 und Beispiel 3 beschrieben durchgeführt.

Die mit dem Plasmid pKE31 transformierten Zellen des Stamms E. coli DSM 14459 wiesen eine spezifische Aktivität von 17 U/mg BTM auf.

### Beispiel 11

### Bestimmung der enzymatischen Aktivitäten in Gegenwart von 100 mM Kaliumcyanid

Die Anzucht der Zellen und die Bestimmung der Aktivitäten in Gegenwart von 100 mM Kaliumcyanid wurden wie in Beispiel 9 und Beispiel 4 beschrieben durchgeführt.

Die mit dem Plasmid pKE31 transformierten Zellen des Stamms E. coli DSM 14459 wiesen eine spezifische Aktivität von 11 U/mg BTM auf.

## Patentansprüche

1. Isolierte Polynukleotide, die für Polypeptide mit den Aminosäuresequenzen kodieren, die zu 97 bis 100 % identisch sind mit den in den Sequenzen SEQ ID NO:2 oder 3 enthaltenden Aminosäuresequenzen, wobei dei Polypeptide die Aktivität einer cyanidtoleranten Nitrilhydratase besitzen.

2. Polynukleotide gemäss Anspruch 1, ausgewählt aus der Gruppe:
a) Polynukleotide, enthaltend die Nukleotidsequenz SEQ ID NO:1 oder dazu komplementäre Nukleotidsequenzen,
b) Polynukleotide enthaltend Nukleotidsequenzen, die den Sequenzen aus a) im Rahmen der Degeneriertheit des genetischen Codes entsprechen,
c) Polynukleotide enthaltend Nukleotidsequenzen gemäss a), die funktionsneutrale Sinnmutationen enthalten,
d) Polynukleotide, die mit den komplementären Sequenzen aus a) unter stringenten Bedingungen hybridisieren, wobei unter stringenten Bedingungen das Waschen in 0,1xSSC bei einer Temperatur von 50 bis 68°C verstanden wird,
wobei die Polynukleotide für eine cyanidtolerante Nitrilhydratase kodieren.

3. Polypeptide, enthaltend Aminsäuresequenzen, die zu 97% bis 100 % identisch sind mit den Sequenzen SEQ ID NO : 2 oder 3.

4. Polypetide mit der Aktivität von cyanidtoleranten Nitrilhydratasen gemäß Anspruch 3, deren Restaktivität nach der Umsetzung von Methacrylnitril in Gegenwart von 20 mM (mM=mmol/l) Cyanidionen bei 20°C nach 30 min. mindestens 90 % der Restaktivität desselben Enzyms beträgt, wenn diese unter ansonsten denselben Bedingungen in Abwesenheit von Cyanidionen für die Umsetzung eingesetzt wurde.

5. Vektoren, enhaltend ein Polynukleotid, ausgewählt aus den gemäss den Ansprüchen 1 oder 2.

6. Wirtszelle, transformiert oder transfektiert durch die Einführung eines Polynukleotids gemäss einem oder mehreren der Ansprüche 1 oder 2.

7. Wirtszelle, transformiert durch die Einführung eines Vektors gemäss Anspruch 6.

8. Verfahren zur enzymatischen Herstellung von Amiden aus Nitrilen, das folgende Schritte aufweist:
a) Umsetzung einer Nitrilgruppen enthaltenden Verbindung mit einem mikrobiellen Enzym (Polypeptid) gemäß Anspruch 4, das Nitrilhydratase-Aktivität aufweist und
b) Abtrennung des gebildeten Amids
wobei man für die Umsetzung des Nitrils zum Amid eine cyanidtolerante Nitrilhydratase gemäß den Ansprüchen 3 oder 4 einsetzt.

9. Verfahren gemäß Anspruch 8, **dadurch gekennzeichnet, dass** man das genannte Enzym produzierende und enthaltende Mikroorganismen gemäss den Ansprüchen 6 oder 7 oder deren Lysat einsetzt.

10. Verfahren gemäß Anspruch 9, **dadurch gekennzeichnet, dass** man ruhende Zelle des Mikroorganismus einsetzt.

11. Verfahren gemäß Anspruch 8, **dadurch gekennzeichnet, dass** man eine gereinigte Nitrilhydratase einsetzt.

12. Verfahren gemäß einem oder mehreren der Ansprüche 8 bis 11, **dadurch gekennzeichnet, dass** das Enzym aus Mikroorganismen der Spezies Pseudomonas marginalis stammt.

13. Verfahren gemäß den Ansprüchen 8 bis 11, **dadurch gekennzeichnet, dass** das Enzym aus eingesetzten Mikroorganismen der Species Pseudomonas marginalis stammt.

14. Verfahren gemäß Anspruch 13, **dadurch gekennzeichnet, dass** die eingesetzten Mikroorganismen unter Nummer DSM 16276 hinterlegt sind.

15. Verfahren gemäß einem oder mehreren der Ansprüche 8 bis 14, **dadurch gekennzeichnet, dass** man Verbindungen der allgemeinen Formel 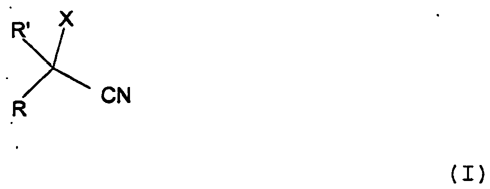
R"-CN (II)
in der bedeuten:
X: OH, H, Alkyl, NH₂;
R: H, gesättigter Alkylrest mit 1 bis 12 C-Atomen, verzweigt oder unverzweigt, gegebenenfalls NH₂-substituiert, ungesättigte Alkylreste mit einer Doppelbindung und 1 bis 12 C-Atomen, verzweigt oder
unverzweigt, Cycloalkylgruppen mit 3 bis 6 C-Atomen,
mit Alkylthiogruppen substituierte Alkylenreste, wobei Alkyl hier einem C₁ bis C₃-Rest
und Alkylen einem zweiwertigen C₃ bis C₈-Rest entspricht,
R': H, Alkyl mit 1 bis 3 C-Atomen,
R": ein- oder zweikerniger ungesättigter Ring, mit 6 bis 12 C-Atomen, gegebenenfalls substituiert mit
einer oder zwei Alkylgruppen (C₁ - C₃), Cl, Br, F. Alkylnitrilrest mit 1 bis 6 C-Atomen
zu den entsprechenden Amiden umsetzt.

16. Verfahren gemäß Anspruch 15, **dadurch gekennzeichnet, dass** man eine Verbindung der allgemeinen Formel(I) in Gegenwart von Blausäure oder einem Salz der Blausäure umsetzt.

17. Verfahren gemäß Anspruch 16, **dadurch gekennzeichnet, dass** man die Umsetzung in Gegenwart einer Anfangskonzentration von mehr als 0,5 mol% Cyanid bis 3 mol% Cyanid, bezogen auf das eingesetzte Nitril, durchführt.

18. Verfahren gemäß einem oder mehreren der Ansprüche 8 bis 17, **dadurch gekennzeichnet, dass** man als Nitril 2-Amino-4-methylthiobutyronitril einsetzt.

19. Verfahren gemäß einem oder mehreren der Ansprüche 8 bis 17, **dadurch gekennzeichnet, dass** man als Nitril 2-Hydroxy-4-methylthiobutyronitril einsetzt, gegebenenfalls enthalten in der Reaktionsmischung aus der Herstellung dieses Nitrils.

20. Verfahren gemäß einem oder mehreren der Ansprüche 8 bis 17, **dadurch gekennzeichnet, dass** man als Nitril 2-Hydroxy-2-methylpropionitril einsetzt.

21. Mikroorganismen der Gattung Pseudomonas, hinterlegt unter der Nummer DSM 16276.

22. Cyanidtolerante Nitrilhydratasen, isoliert aus den Stämmen der Gattung Pseudomonas, hinterlegt unter der Nummer DSM 16276.

## Claims

1. Isolated polynucleotides which code for polypeptides having the amino acid sequences which are 97 to 100% identical to the amino acid sequences contained in the sequences SEQ ID NO: 2 or 3, where the polypeptides have the activity of a cyanide-tolerant nitrile hydratase.

2. Polynucleotides according to Claim 1, selected from the group:
a) polynucleotides comprising the nucleotide sequence SEQ ID NO: 1 or nucleotide sequences complementary thereto,
b) polynucleotides comprising nucleotide sequences which correspond to the sequences from a) within the scope of the degeneracy of the genetic code,
c) polynucleotides comprising nucleotide sequences as in a) which comprise functionally neutral sense mutations,
d) polynucleotides which hybridize with the complementary sequences from a) under stringent conditions, where stringent conditions mean washing in 0.1xSSC at a temperature of from 50 to 68°C,
where the polynucleotides code for a cyanide-tolerant nitrile hydratase.

3. Polypeptides comprising amino acid sequences which are 97% to 100% identical to the sequences SEQ ID NO: 2 or 3.

4. Polypeptides having the activity of cyanide-tolerant nitrile hydratases according to Claim 3, chose remaining activity after conversion of methacrylonitrile in the presence of 20 mM (mM=mmol/l) cyanide ions at 20°C after 30 min is at least 90% of the remaining activity of the same enzyme when it has been employed for the conversion in the absence of cyanide ions under conditions which are otherwise the same.

5. Vectors comprising a polynucleotide selected from those claimed in Claims 1 or 2.

6. Host cell transformed or transfected by the introduction of a polynucleotide according to one or more of Claims 1 or 2.

7. Host cell transformed by the introduction of a vector according to Claim 6.

8. Process for the enzymatic preparation of amides from nitriles, which comprises the hollowing steps:
a) conversion of a compound comprising nitrile groups using a microbial enzyme (polypeptide) according to Claim 4 which has nitrile hydratase activity and
b) removal of the amide formed,
employing a cyanide-tolerant nitrile hydratase according to Claims 3 or 4 for the conversion of the nitrile to the amide.

9. Process according to Claim 8, **characterized in that** microorganisms producing and comprising said enzyme according to Claims 6 or 7, or the lysate thereof, is/are employed.

10. Process according to Claim 9, **characterized in that** resting cells of the microorganism are employed.

11. Process according to Claim 8, **characterized in that** a purified nitrile hydratase is employed.

12. Process according to one or more of Claims 8 to 11, **characterized in that** the enzyme is derived from microorganisms of the species Pseudomonas marginalis.

13. Process according to Claims 8 to 11, **characterized in that** the enzyme is derived from employed microorganisms of the species Pseudonomas marginalis.

14. Process according to Claim 13, **characterized in that** the employed microorganisms are deposited under the number DSM 16276.

15. Process according to one or more of Claims 8 to 14, **characterized in that** compounds of the general formula 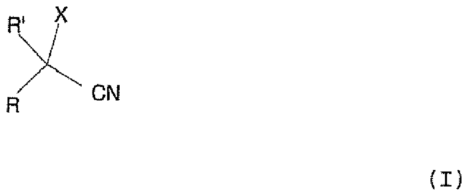
R"-CN (II)
in which the meanings are:
X: OH, H, alkyl, NH₂;
R: H, saturated alkyl radical having 1 to 12 C atoms, branched or unbranched, optionally NH₂-substituted,
unsaturated alkyl radicals having a double bond and 1 to 12 C atoms, branched or unbranched, cycloalkyl groups having 3 to 6 C atoms,
alkylene radicals substituted by alkylthio groups, where alkyl here corresponds to a C₁ to C₃ radical, and alkylene corresponds to a divalent C₃ to C₈ radical,
R': H, alkyl having 1 to 3 C atoms,
R": mono- or binuclear unsaturated ring having 6 to 12 C atoms, optionally substituted by one or two alkyl groups (C₁-C₃), Cl, Br, F, alkyl nitrile radical having 1 to 6 C atoms,
are converted to the corresponding amides.

16. Process according to Claim 15, **characterized in that** a compound of the general formula (I) is converted in the presence of hydrocyanic acid or a salt of hydrocyanic acid.

17. Process according to Claim 16, **characterized in that** the conversion is carried out in the presence of an initial concentration of more than 0.5 mol% cyanide to 3 mol% cyanide, based on the nitrile employed.

18. Process according to one or more of Claims 8 to 17, **characterized in that** 2-amino-4-methylthiobutyronitrile is employed as nitrile.

19. Process according to one or more of Claims 8 to 17, **characterized in that** 2-hydroxy-4-methylthiobutyronitrile, where appropriate present in the reaction mixture from the preparation of this nitrile, is employed as nitrile.

20. Process according to one or more of Claims 8 to 17, **characterized in that** 2-hydroxy-2-methylpropionitrile is employed as nitrile.

21. Microorganisms of the genus Pseudomonas deposited under the number DSM 16276.

22. Cyanide-tolerant nitrile hydratases isolated from the strains of the genus Pseudomonas deposited under the number DSM 16276.

## Revendications

1. Polynucléotides isolés qui codent pour des polypeptides ayant des séquences d'acides aminés qui ont une identité de 97 à 100 % avec les séquences d'acides aminés contenues dans les séquences SEQ ID N°2 ou 3, les polypeptides possédant l'activité d'une nitrile hydratase tolérante aux cyanures.

2. Polynucléotides selon la revendication 1, choisis dans le groupe consistant en :
a) les polynucléotides contenant la séquence nucléotidique SEQ ID N°1 ou des séquences nucléotidiques complémentaires de cette dernière,
b) les polynucléotides contenant des séquences nucléotidiques qui correspondent aux séquences de a) dans le cadre de la dégénérescence du code génétique,
c) les polynucléotides contenant des séquences nucléotidiques selon a), qui contiennent des mutations sens fonctionnellement neutres,
d) les polynucléotides qui s'hybrident aux séquences complémentaires de a) dans des conditions stringentes, auquel cas on entend par conditions stringentes le lavage dans 0,1xSSC à une température de 50 à 68°C,
les polynucléotides codant pour une nitrile hydratase tolérante aux cyanures.

3. Polypeptides contenant des séquences d'acides aminés qui ont une identité de 97 à 100 % avec les séquences SEQ ID N°2 ou 3.

4. Polypeptides ayant l'activité de nitrile hydratases tolérantes aux cyanures selon la revendication 3, dont l'activité résiduelle, après conversion du méthacrylonitrile en présence de 20 mM (mM = mmol/l) d'ions cyanure à 20°C au bout de 30 min est d'au moins 90 % de l'activité résiduelle de cette même enzyme, quand cette dernière avait été utilisée par ailleurs pour la conversion dans les mêmes conditions, en l'absence d'ions cyanure.

5. Vecteurs contenant un polynucléotide choisi parmi les vecteurs selon les revendications 1 ou 2.

6. Cellule hôte, transformée ou transfectée par l'introduction d'un polynucléotide selon l'une ou plusieurs des revendications 1 ou 2.

7. Cellule hôte, transformée par l'introduction d'un vecteur selon la revendication 6.

8. Procédé de préparation enzymatique d'amides à partir de nitriles, qui comprend les étapes suivantes :
a) conversion d'un composé contenant des groupes nitrile avec une enzyme microbienne (polypeptide) selon la revendication 4 présentant une activité de nitrile hydratase, et
b) séparation de l'amide formé,
dans lequel, pour la conversion du nitrile en amide, on utilise une nitrile hydratase tolérante aux cyanures selon les revendications 3 ou 4.

9. Procédé selon la revendication 8, **caractérisé en ce qu'**on utilise des microorganismes selon les revendications 6 à 7, ou un lysat de ces derniers, produisant et contenant l'enzyme mentionnée.

10. Procédé selon la revendication 9, **caractérisé en ce qu'**on utilise une cellule au repos du microorganisme.

11. Procédé selon la revendication 8, **caractérisé en ce qu'**on utilise une nitrile hydratase purifiée.

12. Procédé selon l'une ou plusieurs des revendications 8 à 11, **caractérisé en ce que** l'enzyme provient de microorganismes de l'espèce Pseudomonas marginalis.

13. Procédé selon les revendications 8 à 11, **caractérisé en ce que** l'enzyme provient de microorganismes utilisés de l'espèce Pseudomonas marginalis.

14. Procédé selon la revendication 13, **caractérisé en ce que** les microorganismes utilisés sont déposés sous le numéro DSM 16276.

15. Procédé selon l'une ou plusieurs des revendications 8 à 14, **caractérisé en ce qu'**on fait réagir, pour obtenir les amides correspondants, des composés de formules 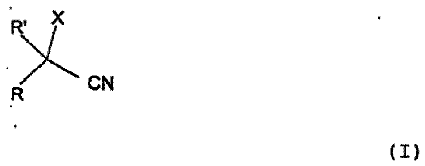
**R"-CN** **(II)**
dans laquelle :
X est OH, H, un radical alkyle, NH₂ ;
R est H, un radical alkyle saturé ayant 1 à 12 atomes de carbone, à chaîne droite ou ramifiée, éventuellement substitué par un ou des substituants NH₂ ;
des radicaux alkyle insaturés ayant une double liaison et 1 à 12 atomes de carbone, à chaîne droite ou ramifiée, des groupes cycloalkyle ayant 3 à 6 atomes de carbone ;
des radicaux alkylène substitués par des groupes alkylthiol, le groupe alkyle correspondant ici à un radical en C₁-C₃ et le groupe alkylène correspondant à un radical divalent en C₃-C₈,
R' est H, un radical alkyle ayant 1 à 3 atomes de carbone,
R" est un noyau mono- ou bicyclique insaturé, ayant 6 à 12 atomes de carbone, éventuellement substitué par un ou deux groupes alkyle en C₁-C₃, Cl, Br, F ; un radical alkylonitrile ayant 1 à 6 atomes de carbone.

16. Procédé selon la revendication 15, **caractérisé en ce qu'**on fait réagir un composé de formule générale (I) en présence d'acide cyanhydrique ou d'un sel de l'acide cyanhydrique.

17. Procédé selon la revendication 16, **caractérisé en ce qu'**on met en oeuvre la réaction en présence d'une concentration initiale de plus de 0,5 % en moles de cyanure à 3 % en moles de cyanure, par rapport au nitrile utilisé.

18. Procédé selon l'une ou plusieurs des revendications 8 à 17, **caractérisé en ce qu'**on utilise en tant que nitrile le 2-amino-4-méthylthiobutyronitrile.

19. Procédé selon l'une ou plusieurs des revendications 8 à 17, **caractérisé en ce qu'**on utilise en tant que nitrile le 2-hydroxy-4-méthylthiobutyronitrile, éventuellement présent dans le mélange réactionnel provenant de la préparation de ce nitrile.

20. Procédé selon l'une ou plusieurs des revendications 8 à 17, **caractérisé en ce qu'**on utilise en tant que nitrile le 2-hydroxy-2-méthylpropionitrile.

21. Microorganismes du genre Pseudomonas, déposés sous le N° DSM 16276.

22. Nitrile hydratases tolérantes aux cyanures, isolées à partir des souches du genre Pseudomonas, déposées sous le numéro DSM 16276.
